(19) Europäisches Patentamt
European Patent Office
Office européen des brevets

(11) Publication number: **0 525 768 A1**

(12) **EUROPEAN PATENT APPLICATION**

(21) Application number: **92113007.6**

(22) Date of filing: **30.07.92**

(51) Int. Cl.$^5$: **C07D 237/16**, C07D 239/42, C07C 255/56, C07C 255/60, C07D 213/64, C07D 213/81, C07D 307/68, A61K 31/395

(30) Priority: **30.07.91 ES 9101777**
**17.02.92 ES 9200333**

(43) Date of publication of application:
**03.02.93 Bulletin 93/05**

(84) Designated Contracting States:
**AT BE CH DE DK ES FR GB GR IT LI LU MC NL PT SE**

(71) Applicant: **J. URIACH & CIA. S.A.**
**Degà Bahi, 59-67**
**E-08026 Barcelona(ES)**

(72) Inventor: **Almansa, Carmen**
**Rossell 498**
**ES-08025 Barcelona(ES)**
Inventor: **González, M Concepci n**
**S.Juan Bosco 56**
**ES-08830 Sant Boi de Llobregat, Barcelon(ES)**
Inventor: **Torres, M Carmen**
**Tramontana 14-16**
**ES-08860 Castelldefels, Barcelona(ES)**
Inventor: **Carceller, Elena**
**Arag n 117**
**ES-08015 Barcelona(ES)**
Inventor: **Bartroli, Javier**
**Vives i Tut 55**
**ES-08034 Barcelona(ES)**

(74) Representative: **Zumstein, Fritz, Dr.**
**Zumstein & Klingseisen Patentanwälte**
**Bräuhausstrasse 4**
**W-8000 München 2(DE)**

(54) **Tetralones with pharmacological activity.**

(57) The present invention relates to new tetralones having the formula **I**:

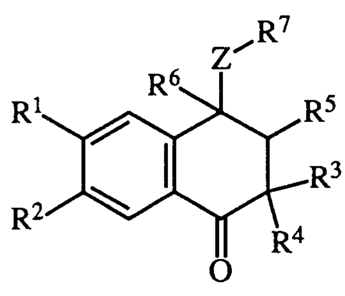

**I**

wherein $R^1$-$R^7$ and Z are as defined in Claim 1. The invention also relates to processes for their preparation and to pharmaceutical compositions containing them. These compounds are antihypertensive and bronchodilating agents.

EP 0 525 768 A1

Field of the invention.

The present invention relates to novel tetralones with pharmacological activity. The invention also relates to a process for their preparation, to pharmaceutical compositions containing them and to their use for the manufacture of medicaments useful in the treatment of mammals, including man. Such tetralones have been found to have blood pressure lowering activity, useful in the treatment of hypertension, as well as bronchodilatory activity, useful in the treatment of asthma. They are also indicated in the treatment of other diseases related with the regulation of the smooth muscle contraction in the gastrointestinal, uterus or urinary tract and in the cardiovascular, respiratory or cerebrovascular systems. Such disorders include angina, congestive heart failure, incontinence, irritable bowel syndrome and epilepsy.

Description of the Prior Art.

Several tetralones having antihypertensive activity have been described in the literature, all of them different from the compounds included in the present invention.

Our patent application EP 489300 discloses certain tetralones with antihypertensive activity of general formula:

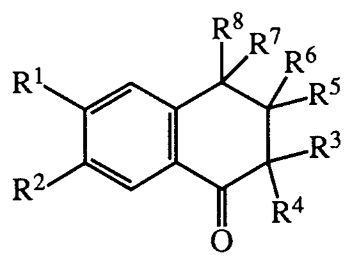

wherein $R^8$ represents an optionally substituted 1,2-dihydro-2-oxo-1-pyridyl (1H-2-Pyridon-1-yl), 1,6-dihydro-6-oxo-1-pyridazinyl (1H-6-Pyridazinon-1-yl), 1,2-dihydro-2-oxo-1-pyrimidinyl (1H-2-Pyrimidinon-1-yl), 1,6-dihydro-6-oxo-1-pyrimidinyl (1H-6-Pyrimidinon-1-yl), 1,2-dihydro-2-oxo-1-pyrazinyl (1H-2-Pyrazinon-1-yl), 1,2-dihydro-2-thioxo-1-pyridyl (1H-2-Thiopyridon-1-yl), 2,3-dihydro-1-oxo-1H-isoindol-2-yl, 1-oxo-1,2,3,4-tetrahydroisoquinol-2-yl, 2-oxo-1-pyrrolidinyl (2-Pyrrolidinon-1-yl), 2-oxo-1-pyperidinyl (2-Pyperidinon-1-yl), 2-thioxo-1-pyrrolidinyl (2-Thiopyrrolidinon-1-yl), or 2-thioxo-1-pyperidinyl (2-Thiopyperidinon-1-yl) radical.

The present invention describes new compounds structurally related to the ones described therein, where the nature of the substituent in position 4 of the tetralone ring has been modified.

Description of the invention.

The present invention relates to new tetralones of general formula **I**:

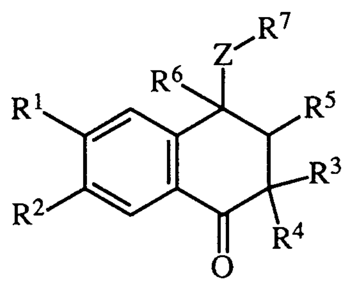

**I**

wherein:

**$R^1$** and **$R^2$** represent hydrogen, $C_{1-4}$ alkyl, hydroxyl, $C_{1-4}$ alkoxy, formyl, $C_{1-4}$ alkylcarbonyl, $C_{1-4}$ alkylthiocarbonyl, carboxyl, $C_{1-4}$ alkoxycarbonyl, $C_{1-4}$ alkoxythiocarbonyl, $C_{1-4}$ alkylcarbonyloxy, $C_{1-4}$ alkylthiocarbonyloxy, hydroxy($C_{1-4}$) alkyl, mercapto-($C_{1-4}$) alkyl, perfluoro($C_{1-4}$)alkyl, nitro, amino, cyano,

halogen, trifluoromethoxy, ethynyl, trimethylsilylethynyl, $C_{1-4}$ alkylsulfinyl, arylsulfinyl, $C_{1-4}$ alkylsulfonyl, arylsulfonyl, $C_{1-4}$ alkoxysulfinyl, $C_{1-4}$ alkoxysulfonyl, $C_{1-4}$ alkylcarbonylamino, $C_{1-4}$ alkoxycarbonylamino, aminosulfinyl, aminosulfonyl, aminocarbonyl, aminothiocarbonyl, $C_{1-4}$ alkylsulfinylamino, $C_{1-4}$ alkylsulfonylamino, $C_{1-4}$ alkoxysulfinylamino, $C_{1-4}$ alkoxysulfonylamino, ($C_{1-4}$ alkyl)carbonyl($C_{1-4}$ alkyl), nitro-($C_{1-4}$ alkyl), cyano($C_{1-4}$ alkyl), ($C_{1-4}$ alkyl)C(=NOH), ($C_{1-4}$ alkyl)C(=$NNH_2$) or ($C_{1-4}$ alkoxy)C(=NH), being the above amino groups optionally substituted by one or two $C_{1-4}$ alkyl groups;

**$R^3$** is hydrogen or $C_{1-4}$ alkyl; **$R^4$** is $C_{1-4}$ alkyl, or $R^3$ and $R^4$ together form a $C_{2-5}$ polymethylene chain;

either **$R^5$** is hydroxyl, acetoxy or formyloxy and **$R^6$** is hydrogen; or $R^5$ and $R^6$ together form a bond;

Z represents O or $NR^8$ such that

a) when Z represents O, then **$R^7$** is a group $R^9$, wherein $R^9$ is $C_{3-6}$ cycloalkyl, $C_{3-6}$ cycloalkenyl, phenyl or heteroaryl, all of them being optionally substituted by one or two halogen atoms and/or one or two $C_{1-6}$ alkyl, $C_{2-4}$ alkenylmethyl, $C_{2-4}$ alkynylmethyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$alkoxycarbonyl, hydroxy, oxo, nitro, cyano, trifluoromethyl, carboxyl, mercapto, amino or amino substituted by one or two $C_{1-4}$ alkyl or aryl groups;

b) when Z represents $NR^8$, then **$R^7$** represents a radical $R^9$ or C(=X)$R^{10}$, where:

$R^8$ means hydrogen, $C_{1-6}$ alkyl, $C_{1-6}$ alkoxy, aryloxy, arylmethyloxy, $C_{1-6}$ alkylcarbonyloxy, arylcarbonyloxy, $C_{1-6}$ alkylcarbonylamino or arylcarbonylamino;

$R^9$ has the previously defined meaning;

X represents O, S or NCN; and

$R^{10}$ is hydrogen, $C_{1-6}$ alkyl (which may be optionally substituted by an hydroxy, $C_{1-6}$ alkoxy or $C_{1-6}$ alkoxycarbonyl group), $C_{2-6}$ alkenyl, $C_{3-6}$ cycloalkyl, $C_{3-6}$ cycloalkenyl, phenyl or heteroaryl, these last two radicals being optionally substituted by a group chosen from halogen, $C_{1-6}$ alkyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups;

and the salts thereof.

The invention also provides the use of at least one compound of formula **I** or a pharmaceutically acceptable salt thereof for the manufacture of a medicament for the treatment and/or prevention of the diseases related with the regulation of the smooth muscle contraction at the cardiovascular, respiratory and cerebrovascular systems, and at the gastrointestinal, urinary and uterus tracts, and particularly for the treatment and/or prevention of hypertension and asthma in mammals, including man.

The invention further provides a pharmaceutical composition comprising an effective amount of at least one compound of formula **I** or a pharmaceutically acceptable salt thereof in admixture with a pharmaceutically acceptable excipient.

The invention still further provides processes for preparing the compounds of formula **I**, which in general terms comprise:

(a) when in a compound of general formula **I**, $R^5$ is OH, $R^6$ is H and Z represents O, reacting a compound of general formula **V**, wherein $R^{1'}$ and $R^{2'}$ are $R^1$ or $R^2$ as defined above or a group or atom convertible thereto, and $R^3$ and $R^4$ have the previously defined meaning,

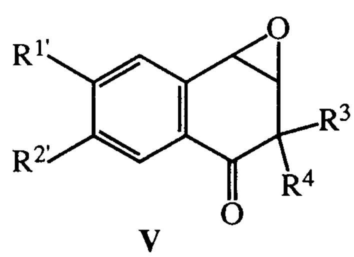

**V**

with a compound of general formula $R^9$-OH (**IX**, wherein $R^9$ has the previously defined meaning) in the presence of a base such as pyridine in a suitable solvent such as a polar solvent, for example ethanol, and optionally, transforming said $R^9$ into other groups $R^9$ by performing standard substitution reactions such as alkylation with an alkyl halide;

(b) when in a compound of general formula **I**, $R^5$ is OH, $R^6$ is H and Z represents $NR^8$, reacting a compound of general formula **V** with a compound of general formula $HNR^8R^9$ (**X**, wherein $R^8$ and $R^9$ have the previously defined meaning) in the presence of a base such as pyridine in a suitable solvent

such as a polar solvent, for example ethanol; or reacting **V** with a compound of general formula $H_2NR^8$ - (**XI**, wherein $R^8$ has the previously defined meaning) in the same experimental conditions, to give a compound of general formula **VI**:

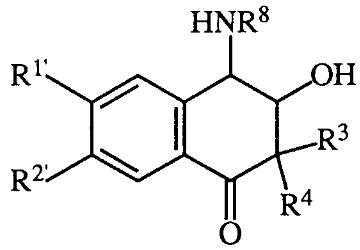

**VI**

wherein $R^{1'}$, $R^{2'}$, $R^3$, $R^4$ and $R^8$ have the previously defined meaning, and then reacting said compound **VI** with a compound of general formula $Y\text{-}R^9$ (**XII**, wherein $R^9$ has the previously defined meaning and Y represents a good leaving group such as an halogen atom or an alkoxy group) in the presence of a base such as triethylamine in a suitable solvent such as a polar solvent, for example ethanol; or reacting a compound of formula **VI** with a compound of general formula $Y\text{-}C(=X)\text{-}R^{10}$ (**XIII**, wherein Y, X and $R^{10}$ have the previously defined meaning) in the presence of a base such as triethylamine in a suitable solvent such as compound **XIII** itself or chloroform, or alternatively, reacting **VI** with a compound of general formula $HO\text{-}C(=X)\text{-}R^{10}$ (**XIV**, wherein X and $R^{10}$ have the previously defined meaning) in the presence of a dehydrating agent such as diciclohexylcarbodiimide in a polar solvent such as dimethylformamide;

(c) in all cases wherein X is sulphur, said compounds may also be obtained by reacting a compound of formula **I** wherein X is oxygen with a thiation reagent such as Lawesson's reagent in a suitable solvent such as toluene;

(d) in all cases wherein $R^5$ is acetoxy and $R^6$ is hydrogen, reacting a compound of formula **I** wherein $R^5$ is hydroxyl and $R^6$ is hydrogen with acetic anhydride in the presence of a base such as pyridine;

(e) in all cases wherein $R^5$ is formyloxy and $R^6$ is hydrogen, reacting a compound of formula **I** wherein $R^5$ is hydroxyl and $R^6$ is hydrogen with formic acid in the presence of a base such as pyridine;

(f) in all cases wherein $R^5$ and $R^6$ together form a bond, reacting a compound of general formula **I** wherein $R^5$ is OH and $R^6$ is hydrogen with a base such as sodium hydride or sodium hydroxyde in a suitable solvent such as toluene or dioxane; or reacting a compound of general formula **I** wherein $R^5$ is acetoxy and $R^6$ is hydrogen with a base such as 1,8-diazabicyclo(5.4.0)undec-7-ene (DBU) in an inert solvent such as toluene;

(g) optionally, interconverting the groups $R^1$, $R^2$, $R^{1'}$ and/or $R^{2'}$ in a compound of formula **I** or any intermediate of formula **II-VI** into other groups $R^1$ and/or $R^2$;

(h) and optionally, reacting a compound of formula **I** with an acid to give its corresponding acid addition salt.

In the compounds of the present invention, a "$C_{1-n}$ alkyl" group means a linear or branched alkyl chain containing from 1 to n carbon atoms. Therefore, in case n is 4 this term includes methyl, ethyl, propyl, isopropyl, butyl, isobutyl, *sec*-butyl or *tert*-butyl, of which methyl, ethyl, propyl, isopropyl, butyl and isobutyl are preferred, methyl and ethyl are more preferred, and methyl is most preferred. When n is 6, it includes, among others, methyl, ethyl, propyl, isopropyl, butyl, isobutyl, *sec*-butyl, *tert*-butyl, pentyl, isopentyl, neopentyl, hexyl, isohexyl,1-methylpentyl, 2-methylpentyl, 3-methylpentyl.

A "$C_{1-n}$ alkoxy" group means a group derived from the union of a $C_{1-n}$ alkyl group to an oxygen atom of an ether functional group. When n is 4, examples include methoxy, ethoxy, propoxy, isopropoxy, butoxy, isobutoxy, *sec*-butoxy and *tert*-butoxy, of which methoxy, ethoxy, propoxy, isopropoxy, butoxy and isobutoxy are preferred, and methoxy is most preferred. When n is 6, examples include methoxy, ethoxy, propoxy, isopropoxy, butoxy, isobutoxy, *sec*-butoxy,*tert*-butoxy, pentyloxy, isopentyloxy, neopentyloxy, hexyloxy, isohexyloxy, 1-methylpentyloxy, 2-methylpentyloxy, 3-methylpentyloxy.

In $R^1$ or $R^2$ a $C_{1-4}$ alkylcarbonyl group means a group derived from the union of a $C_{1-4}$ alkyl group to a carbonyl group. Examples include acetyl, propanoyl, isopropanoyl, butanoyl, and isobutanoyl, of which acetyl and propanoyl are preferred, and acetyl is most preferred.

In $R^1$ or $R^2$ a $C_{1-4}$ alkylthiocarbonyl group means a group derived from the union of a $C_{1-4}$ alkyl group

to a thiocarbonyl group. Examples include thioacetyl, thiopropanoyl, thioisopropanoyl, thiobutanoyl, and thioisobutanoyl, of which thioacetyl and thiopropanoyl are preferred, and thioacetyl is most preferred.

In $R^1$ or $R^2$ a $C_{1-4}$ alkoxycarbonyl group means a group derived from the union of a $C_{1-4}$ alkoxy group, like the above mentioned, to a carbonyl group, and include methoxycarbonyl, ethoxycarbonyl, propoxycarbonyl, isopropoxycarbonyl, butoxycarbonyl, isobutoxycarbonyl, *sec*-butoxycarbonyl and *tert*-butoxycarbonyl, of which methoxycarbonyl, ethoxycarbonyl, propoxycarbonyl, isopropoxycarbonyl, butoxycarbonyl, and isobutoxycarbonyl are preferred, methoxycarbonyl and ethoxycarbonyl are more preferred, and methoxycarbonyl is most preferred.

In $R^1$ or $R^2$ a $C_{1-4}$ alkoxythiocarbonyl group means a group derived from the union of a $C_{1-4}$ alkoxy group, like the above mentioned, to a thiocarbonyl group, and include methoxythiocarbonyl, ethoxythiocarbonyl, propoxythiocarbonyl, isopropoxythiocarbonyl, butoxythiocarbonyl, isobutoxythiocarbonyl, *sec*-butoxythiocarbonyl and *tert*-butoxythiocarbonyl, of which methoxythiocarbonyl, ethoxythiocarbonyl, propoxythiocarbonyl, isopropoxythiocarbonyl, butoxythiocarbonyl, and isobutoxythiocarbonyl are preferred, methoxythiocarbonyl and ethoxythiocarbonyl are more preferred, and methoxythiocarbonyl is most preferred.

In $R^1$ or $R^2$ a $C_{1-4}$ alkylcarbonyloxy group means a group derived from the union of a $C_{1-4}$ alkylcarbonyl group to an oxygen atom. Examples include acetoxy, propanoxy, isopropanoxy, butanoxy, and isobutanoxy, of which acetoxy and propanoxy are preferred, and acetoxy is most preferred.

In $R^1$ or $R^2$ a $C_{1-4}$ alkylthiocarbonyloxy group means a group derived from the union of a $C_{1-4}$ alkylthiocarbonyl group to an oxygen atom. Examples include thioacetoxy, thiopropanoxy, thioisopropanoxy, thiobutanoxy, and thioisobutanoxy, of which thioacetoxy and thiopropanoxy are preferred, and thioacetoxy is most preferred.

In $R^1$ or $R^2$ a hydroxy-$C_{1-4}$ alkyl group means a group resulting from the substitution of one hydrogen atom of the above mentioned "$C_{1-4}$ alkyl" group by an hydroxyl group. Examples include hydroxymethyl, 1-hydroxyethyl, 2-hydroxyethyl, 1-hydroxypropyl, 2-hydroxypropyl, and 3-hydroxypropyl, of which hydroxymethyl, 1-hydroxyethyl and 2-hydroxyethyl are preferred.

In $R^1$ or $R^2$ a mercapto-$C_{1-4}$ alkyl group means a group resulting from the substitution of one hydrogen atom of the above mentioned "$C_{1-4}$ alkyl" group by a mercapto group. Examples include mercaptomethyl, 1-mercaptoethyl, 2-mercaptoethyl, 1-mercaptopropyl, 2-mercaptopropyl, and 3-mercaptopropyl, of which mercaptomethyl, 1-mercaptoethyl and 2-mercaptoethyl are preferred.

In $R^1$ or $R^2$ a perfluoro($C_{1-4}$)alkyl group means a $C_{1-4}$ alkyl group in which all hydrogen atoms have been substituted by fluorine atoms. Examples include trifluoromethyl, pentafluoroethyl, heptafluoropropyl, and nonafluorobutyl, of which trifluoromethyl and pentafluoroethyl are preferred.

In a compound of formula **I**, an amino group may be optionally substituted by one or two $C_{1-4}$ alkyl groups. An amino group substituted by one or two $C_{1-4}$ alkyl groups means a group resulting from the substitution of one or two hydrogen atoms of the amino group by a $C_{1-4}$ alkyl group. When the amino group is substituted by two $C_{1-4}$ alkyl groups, they can be the same or different. Examples include amino, methylamino, dimethylamino, ethylamino, diethylamino, ethylmethylamino, propylamino, dipropylamino, isopropylamino, and diisopropylamino, of which methylamino, dimethylamino, ethylamino and diethylamino are preferred, and methylamino and dimethylamino are most preferred.

The term "halogen" means fluorine, chlorine, bromine or iodine.

In $R^1$ or $R^2$ a $C_{1-4}$ alkylsulfinyl group means a group derived from the union of a $C_{1-4}$ alkyl group to a sulfinyl group. Examples include methylsulfinyl, ethylsulfinyl, propylsulfinyl, isopropylsulfinyl, butylsulfinyl, isobutylsulfinyl, *sec*-butylsulfinyl and *tert*-butylsulfinyl, of which methylsulfinyl, ethylsulfinyl, propylsulfinyl, isopropylsulfinyl, butylsulfinyl and isobutylsulfinyl are preferred, and methylsulfinyl is most preferred.

In a compound of formula **I**, the term "aryl" represents a phenyl group or a phenyl group substituted by a fluorine, chlorine, bromine or iodine atom, or a methyl, hydroxyl, methoxy, cyano or nitro group. Examples include phenyl, 2-methylphenyl, 4-methylphenyl, 4-chlorophenyl, 4-bromophenyl, 4-methoxyphenyl, 2-methoxyphenyl, and 4-cyanophenyl.

In $R^1$ or $R^2$ an arylsulfinyl group means a group derived from the union of an aryl group, like the above mentioned, to a sulfinyl group. Examples include phenylsulfinyl, 2-methylphenylsulfinyl, 4-methylphenylsulfinyl, 4-chlorophenylsulfinyl, 4-bromophenylsulfinyl, 4-methoxyphenylsulfinyl, 2-methoxyphenylsulfinyl, and 4-cyanophenylsulfinyl, of which phenylsulfinyl is preferred.

In $R^1$ or $R^2$ a $C_{1-4}$ alkylsulfonyl group means a group derived from the union of a $C_{1-4}$ alkyl group to a sulfonyl group. Examples include methylsulfonyl, ethylsulfonyl, propylsulfonyl, isopropylsulfonyl, butylsulfonyl, isobutylsulfonyl, *sec*-butylsulfonyl and *tert*-butylsulfonyl, of which methylsulfonyl, ethylsulfonyl, propylsulfonyl, isopropylsulfonyl, butylsulfonyl and isobutylsulfonyl are preferred, and methylsulfonyl is most preferred.

In $R^1$ or $R^2$ an arylsulfonyl group means a group derived from the union of an aryl group, like the above

mentioned, to a sulfonyl group. Examples include phenylsulfonyl, 2-methylphenylsulfonyl, 4-methylphenylsulfonyl, 4-chlorophenylsulfonyl, 4-bromophenylsulfonyl, 4-methoxyphenylsulfonyl, 2-methoxyphenylsulfonyl, and 4-cyanophenylsulfonyl, of which phenylsulfonyl is preferred.

In $R^1$ or $R^2$ a $C_{1-4}$ alkoxysulfinyl group means a group derived from the union of a $C_{1-4}$ alkoxy group to a sulfinyl group. Examples include methoxysulfinyl, ethoxysulfinyl, propoxysulfinyl, isopropoxysulfinyl, butoxysulfinyl, isobutoxysulfinyl, *sec*-butoxysulfinyl and *tert*-butoxysulfinyl, of which methoxysulfinyl, ethoxysulfinyl, propoxysulfinyl, isopropoxysulfinyl, butoxysulfinyl and isobutoxysulfinyl are preferred, and methoxysulfinyl is most preferred.

In $R^1$ or $R^2$ a $C_{1-4}$ alkoxysulfonyl group means a group derived from the union of a $C_{1-4}$ alkoxy group to a sulfonyl group. Examples include methoxysulfonyl, ethoxysulfonyl, propoxysulfonyl, isopropoxysulfonyl, butoxysulfonyl, isobutoxysulfonyl, *sec*-butoxysulfonyl and *tert*-butoxysulfonyl, of which methoxysulfonyl, ethoxysulfonyl, propoxysulfonyl, isopropoxysulfonyl, butoxysulfonyl and isobutoxysulfonyl are preferred, and methoxysulfonyl is most preferred.

In $R^1$ or $R^2$ a $C_{1-4}$ alkylcarbonylamino group means a group derived from the substitution of an hydrogen atom of an amino group, like the above mentioned, by a $C_{1-4}$ alkylcarbonyl group. Examples include acetamido, N-methylacetamido, propanamido, N-methylpropanamido, and isopropanamido, of which acetamido, N-methylacetamido, propanamido and N-methylpropanamido are preferred, and acetamido and N-methylacetamido are most preferred.

In $R^1$ or $R^2$ a $C_{1-4}$ alkoxycarbonylamino group means a group derived from the substitution of an hydrogen atom of an amino group, like the above mentioned, by a $C_{1-4}$ alkoxycarbonyl group. Examples include methoxycarbonylamino, ethoxycarbonylamino, propoxycarbonylamino, isopropoxycarbonylamino, butoxycarbonylamino, and isobutoxycarbonylamino, of which methoxycarbonylamino and ethoxycarbonylamino are preferred, and methoxycarbonylamino is most preferred.

In $R^1$ or $R^2$ an aminosulfinyl group means a group derived from the union of an amino group, like the above mentioned, to a sulfinyl group, and includes, among others, aminosulfinyl, methylaminosulfinyl, dimethylaminosulfinyl, ethylaminosulfinyl, diethylaminosulfinyl, ethylmethylaminosulfinyl, propylaminosulfinyl, dipropylaminosulfinyl, isopropylaminosulfinyl, and diisopropylaminosulfinyl, of which aminosulfinyl, methylaminosulfinyl, dimethylaminosulfinyl, ethylaminosulfinyl, and diethylaminosulfinyl are preferred, and aminosulfinyl, methylaminosulfinyl and dimethylaminosulfinyl are most preferred.

In $R^1$ or $R^2$ an aminosulfonyl group means a group derived from the union of an amino group, like the above mentioned, to a sulfonyl group, and includes, among others, aminosulfonyl, methylaminosulfonyl, dimethylaminosulfonyl, ethylaminosulfonyl, diethylaminosulfonyl, ethylmethylaminosulfonyl, propylaminosulfonyl, dipropylaminosulfonyl, isopropylaminosulfonyl, and diisopropylaminosulfonyl, of which aminosulfonyl, methylaminosulfonyl, dimethylaminosulfonyl, ethylaminosulfonyl, and diethylaminosulfonyl are preferred, and aminosulfonyl, methylaminosulfonyl and dimethylaminosulfonyl are most preferred.

In $R^1$ or $R^2$ an aminocarbonyl group means a group derived from the union of an amino group, like the above mentioned, to a carbonyl group. Examples include aminocarbonyl, methylaminocarbonyl, dimethylaminocarbonyl, ethylaminocarbonyl, diethylaminocarbonyl, ethylmethylaminocarbonyl, propylaminocarbonyl, dipropylaminocarbonyl, isopropylaminocarbonyl, and diisopropylaminocarbonyl, of which aminocarbonyl, methylaminocarbonyl, dimethylaminocarbonyl, ethylaminocarbonyl and diethylaminocarbonyl are preferred, and aminocarbonyl, methylaminocarbonyl and dimethylaminocarbonyl are most preferred.

In $R^1$ or $R^2$ an aminothiocarbonyl group means a group derived from the union of an amino group, like the above mentioned, to a thiocarbonyl group. Examples include aminothiocarbonyl, methylaminothiocarbonyl, dimethylaminothiocarbonyl, ethylaminothiocarbonyl, diethylaminothiocarbonyl, ethylmethylaminothiocarbonyl, propylaminothiocarbonyl, dipropylaminothiocarbonyl, isopropylaminothiocarbonyl, and diisopropylaminothiocarbonyl, of which aminothiocarbonyl, methylaminothiocarbonyl, dimethylaminothiocarbonyl, ethylaminothiocarbonyl and diethylaminothiocarbonyl are preferred, and aminothiocarbonyl, methylaminothiocarbonyl and dimethylaminothiocarbonyl are most preferred.

In $R^1$ or $R^2$ a $C_{1-4}$ alkylsulfinylamino group means a group resulting from the substitution of an hydrogen atom of an amino group, like the above mentioned, by a $C_{1-4}$ alkylsulfinyl group. Examples include methylsulfinylamino, ethylsulfinylamino, propylsulfinylamino, isopropylsulfinylamino, butylsulfinylamino, isobutylsulfinylamino, *sec*-butylsulfinylamino and *tert*-butylsulfinylamino, of which methylsulfinylamino and ethylsulfinylamino are preferred, and methylsulfinylamino is most preferred.

In $R^1$ or $R^2$ a $C_{1-4}$ alkylsulfonylamino group means a group resulting from the substitution of an hydrogen atom of an amino group, like the above mentioned, by a $C_{1-4}$ alkylsulfonyl group. Examples include methylsulfonylamino, ethylsulfonylamino, propylsulfonylamino, isopropylsulfonylamino, butylsulfonylamino, isobutylsulfonylamino, *sec*-butylsulfonylamino and *tert*-butylsulfonylamino, of which methylsulfonylamino and ethylsulfonylamino are preferred, and methylsulfonylamino is most preferred.

In $R^1$ or $R^2$ a $C_{1-4}$ alkoxysulfinylamino group means a group resulting from the substitution of an hydrogen atom of an amino group, like the above mentioned, by a $C_{1-4}$ alkoxysulfinyl group. Examples include methoxysulfinylamino, ethoxysulfinylamino, propoxysulfinylamino, isopropoxysulfinylamino, butoxysulfinylamino, isobutoxysulfinylamino, *sec*-butoxysulfinylamino and *tert*-butoxysulfinylamino, of which methoxysulfinylamino and ethoxysulfinylamino are preferred, and methoxysulfinylamino is most preferred.

In $R^1$ or $R^2$ a $C_{1-4}$ alkoxysulfonylamino group means a group resulting from the substitution of an hydrogen atom of an amino group, like the above mentioned, by a $C_{1-4}$ alkoxysulfonyl group. Examples include methoxysulfonylamino, ethoxysulfonylamino, propoxysulfonylamino, isopropoxysulfonylamino, butoxysulfonylamino, isobutoxysulfonylamino, *sec*-butoxysulfonylamino and *tert*-butoxysulfonylamino, of which methoxysulfonylamino and ethoxysulfonylamino are preferred, and methoxysulfonylamino is most preferred.

In $R^1$ or $R^2$ a ($C_{1-4}$ alkyl)carbonyl($C_{1-4}$ alkyl) group means a group derived from the union of a ($C_{1-4}$ alkyl)carbonyl group, like the above mentioned, to a $C_{1-4}$ alkyl group. Preferred examples are 2-oxopropyl, 2-oxobutyl, 3-oxobutyl and 3-oxopentyl.

In $R^1$ or $R^2$ a nitro-($C_{1-4}$ alkyl) group means a group resulting from the substitution of an hydrogen atom of a $C_{1-4}$ alkyl group by a nitro group. Examples include nitromethyl, 1-nitroethyl, 2-nitroethyl, 1-nitropropyl, 2-nitropropyl, and 3-nitropropyl, of which nitromethyl, 1-nitroethyl and 2-nitroethyl are preferred.

In $R^1$ or $R^2$ a cyano-($C_{1-4}$ alkyl) group means a group resulting from the substitution of an hydrogen atom of a $C_{1-4}$ alkyl group by a cyano group. Examples include cyanomethyl, 1-cyanoethyl, 2-cyanoethyl, 1-cyanopropyl, 2-cyanopropyl, and 3-cyanopropyl, of which cyanomethyl, 1-cyanoethyl and 2-cyanoethyl are preferred.

Examples of ($C_{1-4}$ alkyl)C(=NOH) include 1-oximinoethyl, 1-oximinopropyl, 1-oximinobutyl, 2-methyl-1-oximinopropyl, and 1-oximinopentyl, of which 1-oximinoethyl and 1-oximinopropyl are preferred, and 1-oximinoethyl is most preferred.

Examples of ($C_{1-4}$ alkyl)C(=$NNH_2$) include 1-hidrazonoethyl, 1-hidrazonopropyl, 1-hidrazonobutyl, 2-methyl-1-hidrazonopropyl, and 1-hidrazonopentyl, of which 1-hidrazonoethyl and 1-hidrazonopropyl are preferred, and 1-hidrazonoethyl is most preferred.

Examples of ($C_{1-4}$ alkoxy)C(=NH) include methyl imidate, ethyl imidate, propyl imidate, isopropyl imidate, and butyl imidate, of which methyl imidate and ethyl imidate are preferred, and methyl imidate is most preferred.

In a compound of formula **I**, $R^3$ and $R^4$ are preferred to be both $C_{1-4}$ alkyl, more preferably methyl or ethyl, and most preferably methyl.

In a compound of formula **I**, $R^5$ is preferred to be hydroxyl, in which case $R^6$ is hydrogen.

In $R^8$, $R^9$ and $R^{10}$, the definitions of the different meanings for each substituent are those as mentioned above in connection with $R^1$ and $R^2$.

Besides, in a compound of formula **I**, a "$C_{2-n}$ alkenyl" group means a linear or branched alkyl chain containing from 2 to n carbon atoms and which further contains one or more double bonds. When n is 4, examples include ethenyl, 1-propenyl, 2-propenyl, isopropenyl, 1-butenyl, 2-butenyl, 3-butenyl and 1,3-butadienyl. When n is 6, examples include ethenyl, 1-propenyl, 2-propenyl, isopropenyl, 1-butenyl, 2-butenyl, 3-butenyl, 1,3-butadienyl, 1-pentenyl, 2-pentenyl, 3-pentenyl, 4-pentenyl, 1,3-pentendienyl, 2,4-pentendienyl, 1-hexenyl, 2-hexenyl, 3-hexenyl, 4-hexenyl and 5-hexenyl.

The term "$C_{2-4}$ alkynyl" means a linear or branched alkyl chain containing from 2 to 4 carbon atoms and which further contains one or more triple bonds. Examples include ethynyl, 2-propynyl, 2-butynyl, 3-butynyl.

The term "$C_{2-4}$ alkenylmethyl" means a group resulting from the substitution of an hydrogen atom of a methyl group by a $C_{2-4}$ alkenyl group, like the above mentioned.

The term "$C_{2-4}$ alkynylmethyl" means a group resulting from the substitution of an hydrogen atom of a methyl group by a $C_{2-4}$ alkynyl group, like the above mentioned.

Furthermore, an "aryloxy" group means a group derived from the union of an aryl group, like the above mentioned, to an oxygen atom of an ether functional group. An "arylmethyloxy" group means a group derived from the union of an aryl group, like the above mentioned, to a methyloxy group. An "arylcarbonyloxy" group means a group derived from the union of an arylcarbonyl group to an oxygen atom. An "arylcarbonylamino" group means a group derived from the substitution of an hydrogen atom of an amino group by an arylcarbonyl group.

In a compound of formula **I**, a "$C_{3-6}$ cycloalkyl" group means cyclopropyl, cyclobutyl, cyclopentyl and cyclohexyl. They may be optionally substituted, as mentioned above in pages 2 and 3.

In a compound of formula **I**, a "$C_{3-6}$ cycloalkenyl" group means a $C_{3-6}$ cycloalkyl group like the above mentioned which further contains one or more double bonds. They may be optionally substituted, as

mentioned above in pages 2 and 3.

In a compound of formula **I**, an "heteroaryl" group means any radical from a 5- or 6-membered aromatic heterocycle which contains one ring heteroatom chosen from N, O or S or two ring heteroatoms chosen from the pairs N/N, N/O or N/S. Examples of said heterocycles include pyridine, pyridazine, pyrimidine, pyrazine, furane, pyrrole, thiophene, thiazole and imidazole, all of them being optionally substituted as mentioned above in pages 2 and 3. Examples of said optionally substituted heteroaryl groups include: 2-, 3-, or 4-pyridyl; 3-, 4-, 5- or 6-hydroxy-2-pyridyl; 3-, 4-, 5- or 6-acetoxy-2-pyridyl; 3-, 4-, 5- or 6-amino-2-pyridyl; 3-, 4-, 5- or 6-methoxy-2-pyridyl; 3-, 4-, 5- or 6-methyl-2-pyridyl; 3-, 4-, 5- or 6-mercapto-2-pyridyl; 2-, 4- or 5-hydroxy-3-pyridyl; 2-, 4- or 5-acetoxy-3-pyridyl; 2-, 4- or 5-amino-3-pyridyl; 2-, 4- or 5-methoxy-3-pyridyl; 2-, 4- or 5-methyl-3-pyridyl; 2-, 4- or 5-mercapto-3-pyridyl; 3-hydroxy-4-pyridyl; 2-hydroxy-4-pyridyl (1,2-dihydro-2-oxo-4-pyridyl); 2- or 3-acetoxy-4-pyridyl; 2- or 3-amino-4-pyridyl; 2- or 3-methoxy-4-pyridyl; 2-or 3-methyl-4-pyridyl; 2- or 3-mercapto-4-pyridyl; 2-hydroxy-5-pyridyl; 2-acetoxy-5-pyridyl; 2-amino-5-pyridyl; 2-methoxy-5-pyridyl; 2-methyl-5-pyridyl; 2-mercapto-5-pyridyl; 1,2-dihydro-1-methyl-2-oxo-3, 4-, 5- or 6-pyridyl; 1,2-dihydro-1-ethyl-2-oxo-3, 4-, 5- or 6-pyridyl; 4- or 5-hydroxy-3-pyridazinyl or 6-hydroxy-3-pyridazinyl (1,6-dihydro-6-oxo-3-pyridazinyl); 4-, 5- or 6-acetoxy-3-pyridaziniyl; 4-, 5- or 6-methoxy-3-pyridazinyl; 4-, 5- or 6-amino-3-pyridazinyl; 4-, 5- or 6-acetoxy-3-pyridazinyl; 4-, 5- or 6-methyl-3-pyridazinyl; 4-, 5- or 6-mercapto-3-pyridazinyl; 3-, 5-, or 6-hydroxy-4-pyridazinyl; 3-, 5-, or 6-acetoxy-4-pyridazinyl; 3-, 5-, or 6-methoxy-4-pyridazinyl; 3-, 5-, or 6-amino-4-pyridazinyl; 3-, 5-, or 6-mercapto-4-pyridazinyl; 1,6-dihydro-1-methyl-6-oxo-3-, 4- or 5-pyridazinyl; 1,6-dihydro-1-ethyl-6-oxo-3-, 4- or 5-pyridazinyl; 1,6-dihydro-1-propyl-6-oxo-3-, 4- or 5-pyridazinyl; 1,6-dihydro-1-ethenyl-6-oxo-3-, 4- or 5-pyridazinyl; 1,6-dihydro-1-allyl-6-oxo-3-, 4- or 5-pyridazinyl; 1,6-dihydro-1-phenyl-6-oxo-3-, 4- or 5-pyridazinyl; 1,6-dihydro-1-propargyl-6-oxo-3-, 4- or 5-pyridazinyl; 4- or 5-hydroxy-2-pyrimidinyl; 4- or 5-acetoxy-2-pyrimidinyl; 4- or 5-methoxy-2-pyrimidinyl; 4- or 5-amino-2-pyrimidinyl; 4- or 5-mercapto-2-pyrimidinyl; 2-, 5- or 6-hydroxy-4-pyrimidinyl; 2-, 5- or 6-acetoxy-4-pyrimidinyl;2-, 5- or 6-methoxy-4-pyrimidinyl; 2-, 5- or 6-amino-4-pyrimidinyl; 2-, 5- or 6-mercapto-4-pyrimidinyl; 2- or 4-hydroxy-5-pyrimidinyl; 2- or 4-acetoxy-5-pyrimidinyl; 2- or 4-methoxy-5-pyrimidinyl; 2- or 4-amino-5-pyrimidinyl; 2- or 4-mercapto-5-pyrimidinyl; 3-, 5- or 6-hydroxy-2-pyrazinyl; 3-, 5- or 6-acetoxy-2-pyrazinyl; 3-, 5- or 6-methoxy-2-pyrazinyl; 3-, 5- or 6-amino-2-pyrazinyl; 3-, 5- or 6-mercapto-2-pyrazinyl.

Preferred embodiments of the present invention are those compounds of formula **Ia**

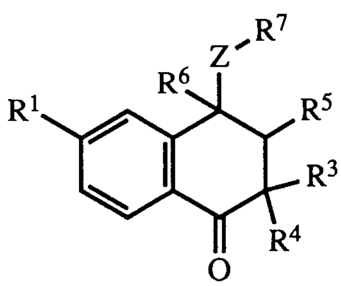

**Ia**

wherein $R^1$, $R^3$, $R^4$, $R^5$, $R^6$, $R^7$ and Z have the previously defined meaning.

More preferred embodiments of the present invention are those compounds of formula **Ib**

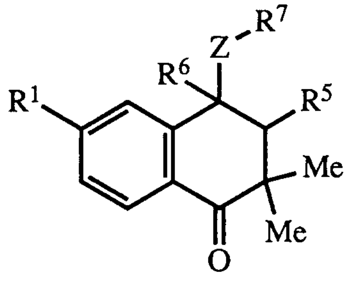

**Ib**

wherein $R^1$, $R^5$, $R^6$, $R^7$ and Z have the previously defined meaning.

Most preferred embodiments of the present invention are those compounds of formula **Ic** and **Id**

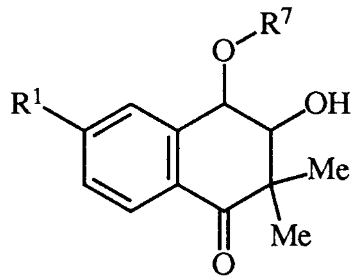

**Ic**

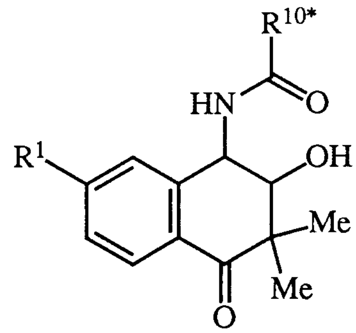

**Id**

wherein $R^1$ and $R^7$ have the previously defined meaning, and $R^{10*}$ means phenyl or heteroaryl, both optionally substituted by a group chosen from halogen, $C_{1-6}$ alkyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups.

The formulae of some specific examples are represented below, together with the number corresponding to the example in which their preparation is described:

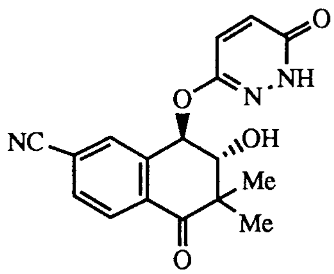
1

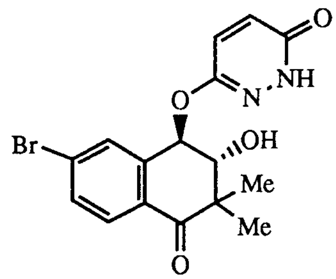
8

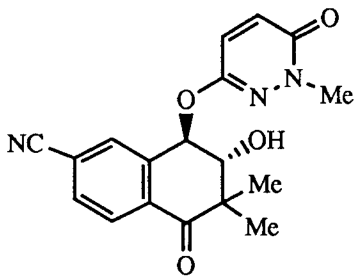
2 (rac)
3 (-)
4 (+)

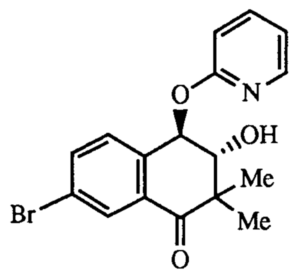
9

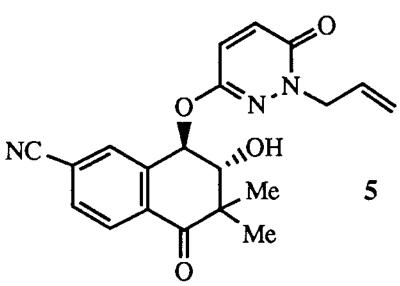
5

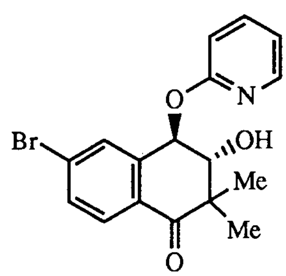
10

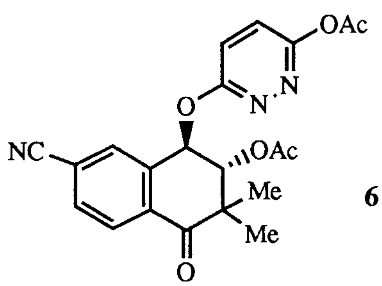
6

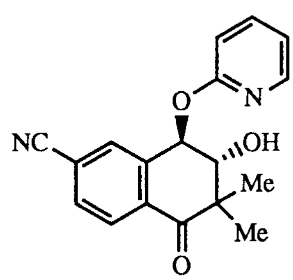
11

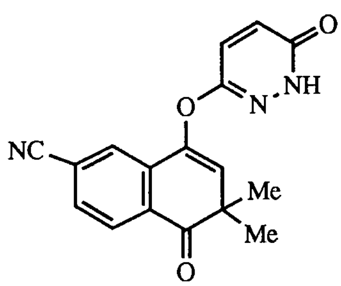
7

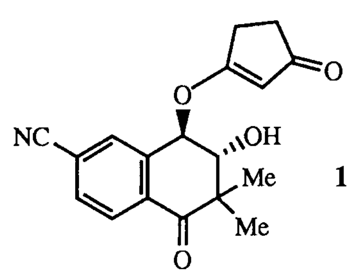
12

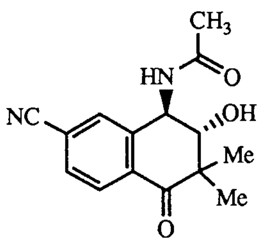

13

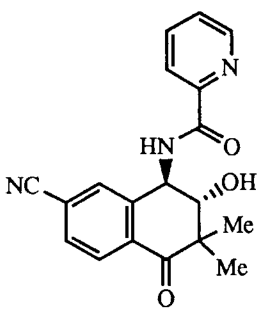

14

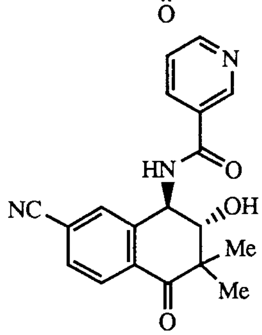

15

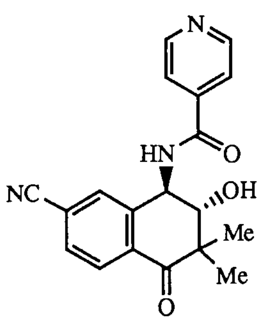

16

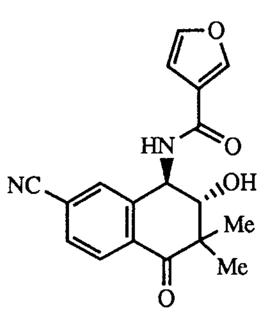

17

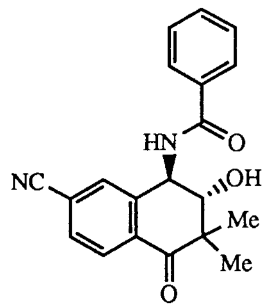

18

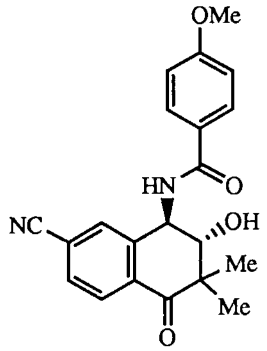

19

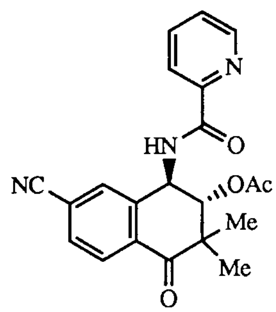

20

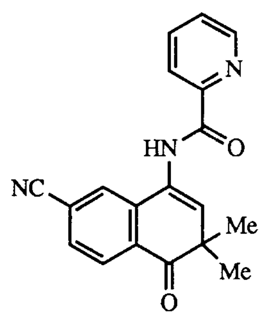

21

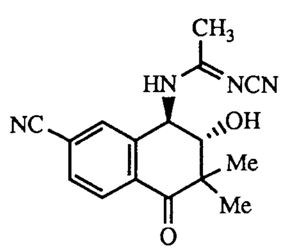

22

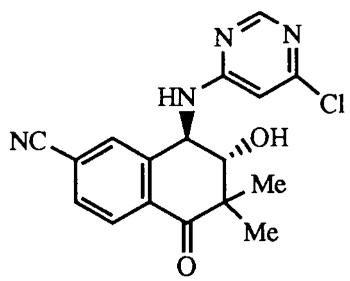
23

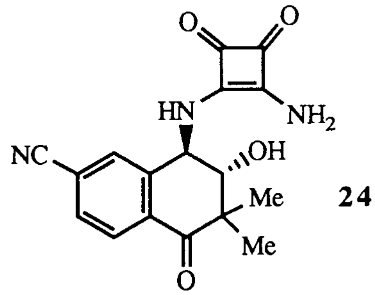
24

Some of the compounds of the present invention contain one or more basic nitrogen atoms and, consequently, they can form salts, which are also included in the present invention. There is no limitation on the nature of these salts but pharmaceutically acceptable ones are preferred. Examples of these salts include: salts with an inorganic acid such as hydrochloric acid, hydrobromic acid, hydriodic acid, nitric acid, perchloric acid, sulfuric acid or phosphoric acid; and salts with an organic acid, such as methanesulfonic acid, trifluoromethanesulfonic acid, ethanesulfonic acid, benzenesulfonic acid, *p*-toluenesulfonic acid, fumaric acid, oxalic acid or maleic acid.

The compounds of the present invention can exist as different diastereoisomers and/or optical isomers because the carbons in positions 3 and 4 of the aromatic ring, provided that there is not a double bond between them, are chiral and in some cases there is an additional chiral center. Diastereoisomers can be separated by conventional techniques such as chromatography or fractional crystallization. The optical isomers can be resolved using any of the conventional optical resolution techniques to give optically pure isomers. Such a resolution can be performed in any chiral synthetic intermediate as well as in the products of general formula **I**. The optical resolution techniques include separation by chromatography on a chiral phase or formation of a diastereoisomeric pair, resolution and subsequent recovery of the two enantiomers. When the chiral intermediate contains a nitrogen atom, a diastereoisomeric salt with a chiral acid such as tartaric acid or camphorsulfonic acid can be formed. Alternatively, the hydroxyl group present at position 3 of the tetralone ring can be reacted in the presence of a base with either enantiomer of a chiral acid such as camphorsulfonic acid, camphanic acid or ($\alpha$)-methoxy-($\alpha$)-trifluoromethylphenylacetic acid (Mosher's acid), followed by chromatography or recrystallization of the two diastereoisomers and ester hydrolisis. The optically pure isomers can also be individually obtained using enantiospecific synthesis. The present invention covers the individual isomers as well as their mixtures (e.g. racemic mixtures), being irrelevant if they have been obtained by synthesis or have been prepared physically mixing them up.

The invention also provides processes for preparing the compounds of formula **I**. The precise method used for the preparation of a given compound of the present invention may vary depending on its chemical structure. Scheme 1 illustrates the general method for their preparation.

## SCHEME 1

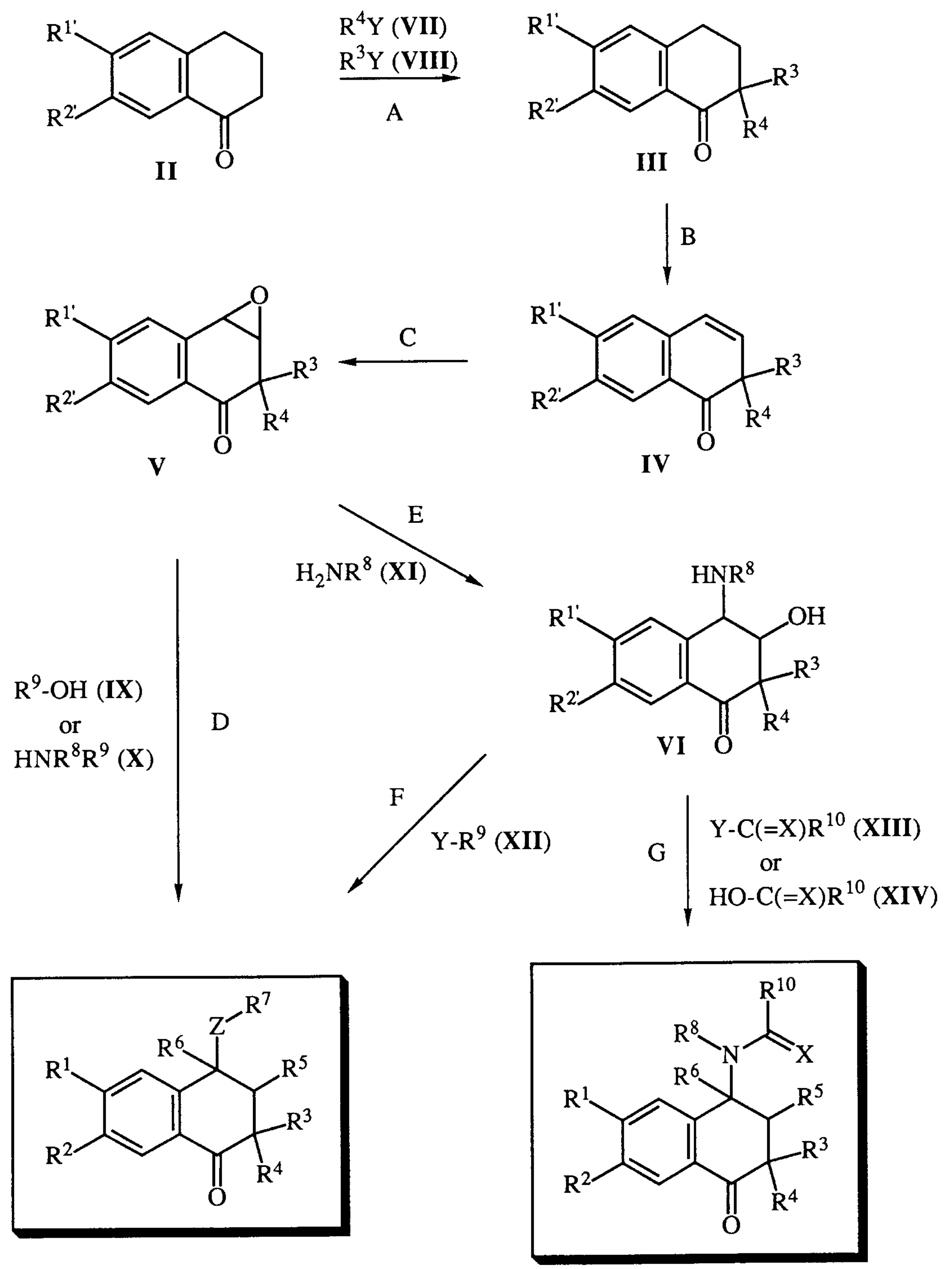


(**I**, Z=O or $NR^8$, $R^7$=$R^9$, $R^5$=OH, $R^6$=H) (**I**, Z=$NR^8$ $R^7$= -C(=X)$R^{10}$, $R^5$=OH, $R^6$=H)

<u>Scheme 1</u>

<u>Wherein:</u>

$R^1$, $R^2$, $R^{1'}$, $R^{2'}$, $R^3$, $R^4$, $R^5$, $R^6$, $R^7$ $R^8$, $R^9$, $R^{10}$ and X have the previously defined meaning; and Y is a good leaving group, such as a chlorine, bromine or iodine atom.

The preparation of the compounds of general formula **I** starts from the compounds of general formula **V**, whose preparation is described in patent application EP 489300 and is summarized in Scheme 1.

The reaction of tetralones **II** (Step A) with an equivalent of a base, such as sodium hydride or butyl lithium, and an alkylating agent of general formula $R^4$-Y (**VII**), in an inert solvent, such as benzene or tetrahydrofuran, at a temperature between room temperature and that of the boiling point of the solvent and during a period of time from 6 to 48 h, leads to the compounds of general formula **III** wherein $R^3$ is hydrogen and $R^4$ is $C_{1-4}$ alkyl. The subsequent alkylation with one more equivalent of base and an alkylating agent of general formula $R^3$-Y (**VIII**) leads to the compounds of general formula **III** wherein $R^3$ and $R^4$ are $C_{1-4}$ alkyl groups. When $R^3$ and $R^4$ are the same, dialkylation can be performed directly, by using two equivalents of base and an excess of alkylating agent in the same experimental conditions described above. In case $R^3$ and $R^4$ together form a $C_{2-5}$ polymethylene chain, the compounds of formula **III** are obtained by alkylation with 2 equivalents of base and an alkylating agent of formula Y-$(CH_2)_p$-Y, wherein Y has the previously defined meaning and p is 2, 3, 4 or 5. The reaction of compounds of general formula **III** -(Step B) with a

brominating agent, such as N-bromosuccinimide or $Br_2$, in an inert solvent, such as carbon tetrachloride, at a temperature between room temperature and that of the boiling point of the solvent and during a reaction time from 4 to 24 h, followed by treatment with a base, such as potassium hydroxide, in an alcoholic solvent, such as ethanol, at a temperature between room temperature and that of the boiling point of the solvent and during a reaction time from 0.5 to 2 h, leads to the compounds of general formula **IV**.

The reaction of compounds of general formula **IV** (Step C) with a peracid, such as *m*-chloroperbenzoic acid, in a suitable solvent, such as methylene chloride, at a reaction temperature from 0 °C to room temperature and during a reaction time from 6 to 24 h leads to the epoxides of general formula **V**.

The reaction of the compounds of general formula **V** (Step D) with a compound of general formula $R^9$-OH (**IX**) or a compound of general formula $HNR^8R^9$ (**X**), wherein $R^8$ and $R^9$ have the previously defined meaning, in the presence of a base, such as pyridine or sodium hydride, in a suitable solvent, such as a polar solvent (e.g. ethanol or tetrahydrofuran), at a temperature from room temperature to that of the boiling point of the solvent and during a reaction time from 4 to 72 h, leads to the compounds of general formula **I** wherein $R^5$ is OH, $R^6$ is hydrogen, Z means respectively O or $NR^8$, being $R^8$ as above defined, and $R^7$ is $R^9$. The nucleophilic attack occurs in the position 4 of epoxide **V**, leading to products with the right regiochemistry and trans configuration, as proved by $^1$H-NMR spectra analysis.

Alternatively, compounds of general formula **I** wherein $R^5$ is OH, $R^6$ is hydrogen, Z is $NR^8$ and $R^7$ is a radical $R^9$, may also be obtained by the following sequence: reaction of a compound of formula **V** with an amine of general formula $H_2NR^8$ (**XI**, wherein $R^8$ has the previously defined meaning) (Step E) in the presence of a base such as pyridine in a suitable solvent, such as a polar solvent (e.g. ethanol), at a temperature between -50 °C and that of the boiling point of the solvent and during a reaction time from 4 h to 7 days, to give a compound of general formula **VI**; and reaction of compound **VI** (Step F) with a compound of general formula Y-$R^9$ (**XII**, wherein $R^9$ and Y have the previously defined meaning) in the presence of a base, such as triethylamine or pyridine, in a suitable solvent, such as a polar solvent (e.g. ethanol), at a temperature between room temperature and that of the boiling point of the solvent and during a reaction time from 4 to 24 h.

Compounds of general formula **I** wherein $R^5$ is OH, $R^6$ is hydrogen, Z is $NR^8$ and $R^7$ is a radical C(=X)$R^{10}$, can be obtained by reaction of a compound of formula **VI** (Step G) with a compound of general formula Y-C(=X)$R^{10}$ (**XIII**, wherein $R^{10}$, X and Y have the previously defined meaning) in a suitable solvent, such as compound **XIII** itself or chloroform, in the presence of a proton scavenger base, such as triethylamine, at a temperature between 0 °C and that of the boiling point of the solvent and during a reaction time from 30 min to 24 h. Alternatively, these compounds may be obtained by reaction of a compound of formula **VI** with a compound of general formula HO-C(=X)$R^{10}$ (**XIV**, wherein $R^{10}$ and X have the previously defined meaning) in the presence of a dehydrating agent, such as dicyclohexylcarbodiimide in the presence of 1-hydroxybenzotriazol, in a suitable solvent; as examples of suitable solvents can be mentioned: dioxane, tetrahydrofuran, acetonitrile, chloroform and N,N-dimethylformamide. The reaction is carried out at a temperature ranging from 0 to 60 °C and during a reaction time from 6 to 24 h.

Compounds of general formula **I** wherein X is sulphur may also be obtained by thiation of the corresponding oxygen derivatives with conventional thiation reagents such as hydrogen sulfide, phosphorous pentasulfide or Lawesson's reagent (*p*-methoxyphenylthiophosphine disulfide) in an apolar inert solvent, such as toluene, at the temperature of the boiling point of the solvent and during a reaction time from 24 to 96 h.

Compounds of general formula **I** wherein $R^5$ is acetoxy and $R^6$ is hydrogen can be obtained by acetylation of the corresponding hydroxy derivatives with acetic anhydride in the presence of a base, such

as pyridine, at room temperature and during a reaction time from 24 to 96 h.

Compounds of general formula **I** wherein $R^5$ is formyloxy and $R^6$ is hydrogen can be obtained by reaction of the corresponding hydroxy derivatives with formic acid in the presence of a base, such as pyridine, and in similar experimental conditions to those mentioned above for the acetylation.

Compounds of general formula **I** wherein $R^5$ and $R^6$ together form a bond can be obtained from the corresponding hydroxy derivatives using conventional reactions of dehydration such as treatment with sodium hydride or sodium hydroxide in an inert solvent, such as tetrahydrofuran or dioxane, at a temperature between room temperature and that of the boiling point of the solvent and during a reaction time from 6 to 48 h, or by treatment of the corresponding acetoxy derivatives with a base, such as 1,8-diazabicyclo(5.4.0)undec-7-ene, in an inert solvent, such as toluene, at a temperature between room temperature and that of the boiling point of the solvent and during a reaction time from 6 to 48 h.

Furthermore, it is also possible to transform the groups $R^1$ and/or $R^2$ in a compound of formula **I** or $R^{1'}$ and/or $R^{2'}$ in one of its synthetic intermediates into other groups $R^1$ and/or $R^2$.

For instance, a cyano group may be transformed into a carboxyl group, into a carbamoyl group, into a methyl carboximidate group, or into a methyl carboxylate group; a bromine atom may be converted into a trifluoromethyl or a pentafluoroethyl group, or into a trimethylsilylethynyl group, which may be subsequently transformed into an ethynyl group; a methoxy group may be transformed into a hydroxyl group, and this may be then converted into a bromine atom; an acetamido group may be alkylated to an N-methylacetamido group, which may be deacetylated to a methylamino group, and this transformed into a N-methylmethanesulfonamide.

Moreover, when in a compound of formula **I** Z is O, it is also possible to transform a group $R^9$ into other groups $R^9$ by performing standard reactions of substitution, such as alkylation with an alkyl halide. These reactions can be carried out under the conventional reaction conditions used in organic chemistry for these kind of transformations.

The compounds of formula **I** may be transformed into their corresponding acid addition salts by treatment with an acid, such as hydrochloric acid, sulphuric acid, nitric acid, oxalic acid or methanesulfonic acid.

The compounds of general formula **I** are useful as antihypertensive agents, as shown by their ability to inhibit the contraction induced by noradrenaline in portal vein isolated from rat, according to test 1, and their ability to lower the blood pressure in hypertense rats, according to test 2.

Test 1: Inhibition of the contraction induced by noradrenaline in portal vein isolated from rat.

Portal vein was extracted from adult male rats (between 200 and 250 g of body weight), which had been stunned by a blow in the head. Portal vein was installed into an isolated organ bath (Letica) containing a physiological saline solution (Hamilton et al., *Br. J. Pharmacol.*, **1986**, 88, 103-111) at 37°C and a gas bubbler (5% $CO_2$ and 95% $O_2$). Contractions were induced by noradrenaline (3μM) and were reverted after thoroughly washing with physiological saline solution. Portal vein contraction was measured with an isometric force transducer and with an initial tension of 1 g. After two contractions with noradrenaline, performed in order to measure the tissue's basal response, the tested compounds were incubated for 30 minutes and a new contraction was induced. The concentration that produces a 50% inhibition ($IC_{50}$) versus the basal response was calculated. The results are shown in table I.

TABLE I

| Compound Nº | $IC_{50}$ (μM) |
|---|---|
| 1 | 8.8 |
| 2 | 2.8 |
| 5 | 14 |
| 8 | 0.8 |
| 11 | 25 |
| 14 | 23 |
| 15 | 2.8 |
| 18 | 10 |
| 19 | 12 |
| 23 | 3.7 |

Test 2: Lowering of the arterial pressure in conscious hypertense rats.

Spontaneously hypertense rats (between 200 and 250 g of body weight) of more than 8 weeks of age have been used. Diastolic and systolic arterial pressure of the rat was measured at the caudal artery using a special sphygnomanometer (Letica 5007 and 5007/4) attached to the animal's tail. To ensure rapid and reliable data, animals were placed in a heating plate at 37°C, with the aim to produce a vasodilatation that ensured a better fixation of the rat tail to the transducer chamber. During the experiment, rats are conscious and fixed to a clamp. The tested products were administered orally. Arterial pressure was measured every 30 minutes and 10 minutes before the administration of the tested compound. The decrease of the arterial pressure was calculated for each compound at a dose of 1 mg/Kg, using 3 animals. The results are shown in table II.

TABLE II

| Compound Nº | Pressure Decrease (mm Hg) ± SEM (1 mg/Kg p.o.) |
|---|---|
| 1 | 116.0 ± 5.9 |
| 2 | 112.0 ± 1.0 |
| 5 | 20.5 ± 0.5 |
| 8 | 46.5 ± 0.3 |
| 11 | 43.0 ± 16.0 |
| 12 | 50.0 ± 16.0 |
| 14 | 71.7 ± 14.2 |
| 15 | 50.0 ± 9.8 |
| 17 | 58.0 ± 17.5 |
| 18 | 31.0 ± 11.9 |
| 19 | 46.7 ± 8.6 |
| 23 | 45.5 ± 11.5 |

Furthermore, we have found that compounds of general formula **I** are bronchodilator agents, according to test 3.

Test 3 -Direct relaxation of the trachea isolated from guinea pig.

This test was performed according to the experimental model described by Emmerson, J. and Mackay, D. (*J. Pharm. Pharmacol.*, **1979**, 31, 798). Tracheas were extracted from male guinea pigs of 400 g of body weight, which had been stunned by a blow in the head. Then, tracheas were cut in zigzag and placed into an isolated organ bath (Letica) containing Krebs-Henseleit solution at 37°C and were bubbled with carbogen (95% $O_2$ and 5% $CO_2$). The relaxation of the trachea was measured using an isometric force transducer. The basal tension was 0.5 g. The tested compounds were accumulatively added to the bath and the effective concentration which produces a 50% of the maximum relaxation ($EC_{50}$) was calculated. The maximum relaxation was considered to be the relaxation induced by isoproterenol at $1x10^{-6}$ M. Results are shown in table III.

TABLE III

| Compound No | $EC_{50}$ (μM) |
|---|---|
| 1 | 5.2 |
| 2 | 0.31 |
| 3 | 0.3 |
| 4 | 4.6 |
| 5 | 3.3 |
| 8 | 15.6 |
| 11 | 5.8 |
| 15 | 4.9 |
| 18 | 2.1 |
| 19 | 0.3 |
| 22 | 1.8 |
| 23 | 4.4 |

Solid compositions according to the present invention for oral administration include compressed tablets, dispersible powders, granules and capsules. In tablets, one or more of the active component(s) is admixed with at least one inert diluent such as lactose, starch, mannitol, microcrystalline cellulose or calcium phosphate; granulating and disintegrating agents for example corn starch, gelatine, microcrystalline cellulose or polyvinylpyrrolidone; and lubricating agents for example magnesium stearate, stearic acid or talc. The tablets may be coated by known techniques to delay disintegration and absorption in the gastrointestinal tract and, thereby, provide a sustained action over a longer period. Gastric film-coated or enteric film-coated can be made with sugar, gelatin, hydroxypropylcellulose, or acrylic resins. Tablets with a sustained action may also be obtained using an excipient which provides regressive osmosis, such as the galacturonic acid polymers. Formulations for oral use may also be presented as hard capsules of absorbable material, such as gelatin, wherein the active ingredient is mixed with an inert solid diluent and lubricating agents, or pasty materials, such as ethoxylated saturated glycerides that could exhibit controlled liberation. Soft gelatin capsules are possible wherein the active ingredient is mixed with water or an oily medium, for example peanut oil, liquid paraffin or olive oil.

Dispersible powders and granules suitable for preparation of a suspension by the addition of water provide the active ingredient in admixture with a dispersing or wetting agent, a suspending agent, such as sodium carboxymethylcellulose, methylcellulose, hydroxypropylmethylcellulose, sodium alginate, polyvinylpyrrolidone, gum tragacanth, xantham gum, gum acacia, and one or more preservatives, such as methyl or *n*-propyl-*p*-hydroxybenzoate. Additional excipients, for example sweetening, flavouring and colouring agents may also be present.

Liquid compositions for oral administration include emulsions, solutions, suspensions, syrups and elixirs containing commonly used inert diluents, such as distilled water, ethanol, sorbitol, glycerol, or propylene glycol. Such compositions may also comprise adjuvants such as wetting agents, suspending agents, sweetening, flavouring, perfuming, preserving agents and buffers.

Other compositions for oral administration include spray compositions, which may be prepared by known methods and which comprise one or more active compound(s). The spray compositions will contain a suitable propellent.

Preparations for injection according to the present invention for parenteral administration include sterile aqueous or non-aqueous solutions, suspensions or emulsions, in a non-toxic parentally-acceptable diluent or solvent. Examples of aqueous solvents or suspending media are distilled water for injection, the Ringer's solution, and isotonic sodium chloride solution. Examples of non-aqueous solvents or suspending media are propylene glycol, polyethylene glycol, vegetable oils such as olive oil, or alcohols such as ethanol. These compositions may also include adjuvants such as wetting, preserving, emulsifying and dispersing agents. They may be sterilized by one of the known methods or manufactured in the form of sterile solid compositions which can be dissolved in sterile water or some other sterile injectable medium immediately before use. When all of the components are sterile, the injectables will maintain the sterility if they are manufactured in sterile environment.

A compound of the invention may also be administered in the form of suppositories for rectal administration of the drug, or as creams, ointments jellies, solutions or suspensions for topical use and pessaries for vaginal administration.

The dosage and frequency of dose may vary depending upon symptoms, age and body weight of the

patient, as well as upon the route of administration, but, in general, the compounds of the invention may be administered orally in a daily dose of from 0.1-100 mg for an adult, preferably a dosage from 2-50 mg, which may be administered either as a single dose or as divided doses.

Following are some representative preparations for tablets, capsules, syrups, aerosols and injectables. They can be prepared following standard procedures and they are useful in the treatment of diseases related with the regulation of the smooth muscle contraction, in the cardiovascular and respiratory systems and in the gastrointestinal, urinary and uterus tracts, and particularly as antihypertensive and bronchodilator agents.

| **Tablets** | |
|---|---|
| Compound of formula **I** | 100 mg |
| Dibasic calcium phosphate | 125 mg |
| Sodium starch glycolate | 10 mg |
| Talc | 12.5 mg |
| Magnesium stearate | 2.5 mg |
| | 250.0 mg |

| **Hard gelatin capsules** | |
|---|---|
| Compound of formula **I** | 100 mg |
| Lactose | 197 mg |
| Magnesium stearate | 3 mg |
| | 300 mg |

| **Syrup** | |
|---|---|
| Compound of formula **I** | 0.4 g |
| Sucrose | 45 g |
| Flavouring agent | 0.2 g |
| Sweetening agent | 0.1 g |
| Water to | 100 mL |

| **Aerosol** | |
|---|---|
| Compound of formula **I** | 4 g |
| Flavouring agent | 0.2 g |
| Propylene glycol to | 100 mL |
| Suitable propellent to | 1 unit |

| **Injectable preparation** | |
|---|---|
| Compound of formula **I** | 100 mg |
| Benzylic alcohol | 0.05 mL |
| Propylene glycol | 1 mL |
| Water to | 5 mL |

The following examples illustrate, but do not limit, the scope of the preparation of the compounds of the present invention.

**PREPARATION 1**

**7-bromo-2,2-dimethyl-1,2,3,4-tetrahydronaphthalen-1-one**

To solution of 9.78 g (39 mmol) of 7-bromo-1,2,3,4-tetrahydronaphthalen-1-one (R. W. Griffin, J. D. Gass, M. A. Berwick, R. S. Shulman, *J.Org.Chem.* **1964**, 29, 2109) and 6.45 mL (105 mmol) of methyl iodide in 74 mL of benzene, were added under argon atmosphere 3.71 g (77 mmol) of 55% sodium hydride. The mixture was stirred at 60 ° C for 5 h and then at reflux overnight. The suspension was poured into methanol and then the solvent was removed. The residue was dissolved in ether and washed with $H_2O$ and $Na_2CO_3$. The organic solution was dried over $MgSO_4$ and the solvent was evaporated, affording a crude that was chromatographed on silica gel eluting with mixtures of hexane-$CH_2Cl_2$ of increasing polarity. 9.90 g of the product were obtained as a colorless oil (yield: 100%).
IR (film) $\nu$: 2957, 2921, 1679, 1583, 1469, 1398, 1302, 1208, 1107, 828 $cm^{-1}$;
$^1$H NMR (80 MHz, $CDCl_3$) $\delta$ (TMS): 8.13 (d, J = 2Hz, 1H, Ar), 7.55 (dd, $J_a$ = 8Hz, $J_b$ = 2Hz, 1H, Ar), 7.09 (d, J = 8Hz, 1H, Ar), 2.92 (t, J = 6.5Hz, 2H, $CH_2$Ar), 1.96 (t, J = 6.5Hz, 2H, $CH_2$), 1.20 (s, 6H, $2CH_3$).

**PREPARATION 2**

**7-bromo-2,2-dimethyl-1,2-dihydronaphthalen-1-one**

To a solution of 9.90 g (39 mmol) of the product obtained in preparation 1 in 212 mL of $CCl_4$, were added under argon atmosphere 9.05 g (51 mmol) of N-bromosuccinimide and 0.42 g of benzoyl peroxide and the mixture was stirred at reflux for 5 h. The imide formed was filtered and the solvent was removed. The residue was treated with 76 mL of 10% KOH in ethanol and then heated at reflux for 1 h. After removal of the solvent, the residue was dissolved in ether, washed with $H_2O$ and $Na_2CO_3$ and dried over $MgSO_4$. The solvent was removed and the residue chromatographed on silica gel eluting with mixtures of hexane-$CH_2Cl_2$ of increasing polarity. 7.08 g of the product were obtained as a colorless oil (yield: 72%).
IR (film) $\nu$: 3051, 3024, 2961, 2919, 1669, 1581, 1471, 1181, 836 $cm^{-1}$;
$^1$H NMR (80 MHz, $CDCl_3$) $\delta$ (TMS): 8.14 (d, J = 2Hz, 1H, Ar), 7.63 (dd, $J_a$ = 8Hz, $J_b$ = 2Hz, 1H, Ar), 7.09 (d, J = 8Hz, 1H, Ar), 6.47 (d, J = 9.6Hz, 1H, CHAr), 6.12 (d, J = 9.6Hz, 1H, CH), 1.27 (s, 6H, $2CH_3$).

**PREPARATION 3**

**7-bromo-2,2-dimethyl-3,4-epoxy-1,2,3,4-tetrahydronaphthalen-1-one**

To a solution of 7.08 g (28 mmol) of the product obtained in preparation 2 in 170 mL of $CH_2Cl_2$, were added 10.76 g (34 mmol) of *m*-chloroperbenzoic acid and the resulting mixture was stirred overnight at room temperature. The resulting solution was washed with $Na_2S_2O_5$ solution and then with $NaHCO_3$ solution, and the organic phase was dried over $MgSO_4$. The solvent was removed, yielding 9.07 g of a crude that was chromatographed on silica gel eluting with hexane-$CH_2Cl_2$ mixtures of increasing polarity. 6.18 g of the title compound of this preparation were obtained as a white solid (yield: 83%).
M.p.: 62-63 ° C;
IR (film) $\nu$: 2977, 2961, 1684, 1586, 1454, 1278, 1174, 897 $cm^{-1}$;
$^1$H NMR (80 MHz, $CDCl_3$) $\delta$ (TMS): 8.05 (d, J = 2Hz, 1H, Ph), 7.69 (dd, $J_a$ = 8Hz, $J_b$ = 2Hz, 1H, Ph), 7.44 (d, J = 8Hz, 1H, Ph), 4.02 (d, J = 4Hz, 1H, CHO), 3.56 (d, J = 4Hz, 1H, CHO), 1.50 (s, 3H, $CH_3$), 1.12 (s, 3H, $CH_3$).

**EXAMPLE 1**

***Trans*-2,2-dimethyl-3-hydroxy-4-(6-hydroxy-3-pyridazinyloxy)-1-oxo-1,2,3,4-tetrahydronaphthalen-6-carbonitrile**

To a solution of 0.5 g (2.3 mmol) of 3,4-epoxy-2,2-dimethyl-1-oxo-1,2,3,4-tetrahydronaphthalen-6-carbonitrile (EP 489300) in 4.2 mL of ethanol, were added 0.25 g (2.3 mmol) of 3,6-dihydroxypyridazine and 0.18 mL (2.3 mmol) of pyridine and the mixture was stirred at reflux under argon atmosphere for 2 days. The solvent was removed and the residue dissolved in $CH_2Cl_2$. After filtration, 0.450 g of the desired product were obtained as a white solid. The filtrate was chromatographed on silica gel eluting with hexane-ethyl acetate mixtures, further yielding 0.050 g of the title compound of this example (yield: 65%).
M.p.: 250-252 ° C;
IR (KBr) $\nu$: 3400-2800, 2231, 1686, 1663, 1594, 1444, 1280, 1056, 979 $cm^{-1}$;

$^1$H NMR (80 MHz, $CDCl_3$-$CD_3OD$) δ (TMS): 8.16 (d, J = 8Hz, 1H, Ar), 7.78 (m, 2H, Ar), 7.22 (d, J = 9.6Hz, 1H, pyr), 7.02 (d, J = 9.6Hz, 1H, pyr), 6.17 (d, J = 8Hz, 1H, CHO), 4.01 (d, J = 8Hz, 1H, CHOH), 3.68 (s, 2H, OH + NH), 1.36 (s, 3H, $CH_3$), 1.25 (s, 3H, $CH_3$).
Analysis Calcd. for $C_{17}H_{15}N_3O_4$: C 62.76%; H 4.65%; N 12.92%. Found: C 62.35%; H 4.69%; N 13.08%.

**EXAMPLE 2**

***Rac-Trans*-2,2-dimethyl-3-hydroxy-4-(6-hydroxy-1-methyl-3-pyridazinyloxy)-1-oxo-1,2,3,4-tetrahydronaphthalen-6-carbonitrile**

To a solution of 0.32 g (0.98 mmol) of the product obtained in example 1 in 15 mL of acetone, were added 0.98 g (7.1 mmol) of potassium carbonate and 0.44 mL (7.1 mmol) of methyl iodide. The resulting mixture was stirred at reflux for 3 h and then the solvent was removed. The residue was dissolved in a mixture of $H_2O$ and $CH_2Cl_2$ and the layers were separated. The aqueous phase was extracted with $CH_2Cl_2$ and the combined organic phases were dried over $MgSO_4$. The solvent was removed, yielding a residue that was chromatographed on silica gel eluting with hexane-ethyl acetate mixtures of increasing polarity. 0.220 g of the title compound of this example were obtained as a white solid (yield: 66%).
M.p.: 129-132 ° C;
IR (KBr) ν: 3600-3000, 2930, 2867, 2231, 1685, 1653, 1573, 1539, 1432, 1291, 1263, 1036, 997, 841 $cm^{-1}$;
$^1$H NMR (80 MHz, $CDCl_3$) δ (TMS): 8.19 (d, J = 8Hz, 1H, Ar), 7.75 (m, 2H, Ar), 7.06 (s, 2H, pyr), 6.19 (d, J = 8.8Hz, 1H, CHO), 4.06 (d, J = 8.8Hz, 1H, CHOH), 3.68 (s, 3H, $CH_3$), 2.0 (broad s., 1H, OH), 1.42 (s, 3H, $CH_3$), 1.28 (s, 3H, $CH_3$).
Analysis Calcd. for $C_{18}H_{17}N_3O_4$. 0.75 $H_2O$: C 61.28%; H 5.25%; N 11.91%. Found: C 61.45%; H 5.45%; N 11.56%.

**EXAMPLE 3**

***(-)-Trans*-2,2-dimethyl-3-hydroxy-4-(6-hydroxy-1-methyl-3-pyridazinyloxy)-1-oxo-1,2,3,4-tetrahydronaphthalen-6-carbonitrile**

**A.- *Trans*-2,2-dimethyl-4-(6-hydroxy-1-methyl-3-pyridazinyloxy)-3-[(S)-α-methoxy-α-(trifluoromethyl)-phenylacetyloxy]-1-oxo-1,2,3,4-tetrahydronaphthalen-6-carbonitrile**

To a solution of 1 g (3 mmol) of the product obtained in example 2, 0.78 g (3.8 mmol) of dicyclohexylcarbodiimide and 0.04 g of dimethylaminopyridine in 8 mL of $CH_2Cl_2$, were added under argon atmosphere 0.68 g (3 mmol) of (S)-(-)-α-methoxy-α-(trifluoromethyl)phenylacetic acid and the mixture was stirred at room temperature for 3 h. The suspension thus obtained was washed with water and saturated $NaHCO_3$ solution and dried over $MgSO_4$. The solvent was removed and the residue was chromatographed several times on silica-gel columns. The most polar fractions afforded 0.362 g of the title compound of this example as a white solid (yield: 22%).
M.p.: 67-73 ° C;
$[\alpha]D^{24}$ = -105.9 ° (0.5, $CHCl_3$);
IR (KBr) ν: 3062, 2942, 2230, 1746, 1693, 1666, 1591, 1428, 1292, 1256, 1167, 1016 $cm^{-1}$;
$^1$H NMR (80 MHz, $CDCl_3$) δ (TMS): 8.18 (d, J = 8Hz, 1H, Ar), 7.8-7.2 (m, 7H, Ar), 6.90 (AB system, J = 9.6Hz, 2H, pyr), 6.25 (d, J = 8Hz, 1H, CHOPyr), 5.67 (d, J = 8Hz, 1H, CHOCO-), 3.64 (s, 3H, OMe), 3.43 (s, 3H, NMe), 1.23 (s, 6H, 2Me).
Analysis Calcd. for $C_{28}H_{24}F_3N_3O_6$. 0.25 $H_2O$: C 59.57%; H 4.34%; N 7.45%. Found: C 59.90%; H4.52%; N 7.27%.

**B.- Title compound.**

To a solution of 0.290 g (0.5 mmol) of the product obtained in example 3A in 1.8 mL of THF, were added 1.8 mL of 1N NaOH and the mixture was stirred overnight. Water and ethyl acetate were added and the layers were separated. The aqueous phase was extracted with ethyl acetate and the combined organic extracts dried over $MgSO_4$. The solvent was removed and the residue was chromatographed on a silica-gel column to give 0.100 g of the title compound of this example (yield: 56 %).
M.p.: 80-85 ° C;
$[\alpha]D^{24}$ = -129.0 ° (0.5, $CHCl_3$);

IR (KBr) ν: 3600-3000, 2930, 2229, 1691, 1657, 1574, 1535, 1430, 1291, 1260, 1035, 840 $cm^{-1}$;
$^1H$ NMR (80 MHz, $CDCl_3$) δ (TMS): 8.18 (d, J=8Hz, 1H, Ar), 7.76 (m, 2H, Ar), 7.07 (s, 2H, pyr), 6.18 (d, J=8Hz, 1H, CHOPyr), 4.05 (d, J=8Hz, 1H, CHOH), 3.68 (s, 3H, NMe), 3.00 (m, 1H, OH), 1.41 (s, 3H, Me), 1.27 (s, 3H, Me).
Analysis Calcd. for $C_{18}H_{17}N_3O_4$. 0.25 $H_2O$. 0.5 AcOEt: C 61.93%; H 5.55%; N 10.84%. Found: C 62.11%; H5.26%; N 10.93%.

**EXAMPLE 4**

**(+)-*Trans*-2,2-dimethyl-3-hydroxy-4-(6-hydroxy-1-methyl-3-pyridazinyloxy)-1-oxo-1,2,3,4-tetrahydronaphthalen-6-carbonitrile**

**A.- *Trans*-2,2-dimethyl-4-(6-hydroxy-1-methyl-3-pyridazinyloxy)-3-[(S)-α-methoxy-α-(trifluoromethyl)-phenylacetyloxyl-1-oxo-1,2,3,4-tetrahydronaphthalen-6-carbonitrile**

The less polar fractions obtained in the chromatography described in example 3A afforded 0.450 g of the title compound of this example as a white solid (yield: 27%).
M.p.: 67-77 °C;
$[\alpha]D^{24}$ = +111.1 ° (0.5, $CHCl_3$);
IR (KBr) ν: 3059, 2939, 2231, 1750, 1692, 1666, 1591, 1428, 1292, 1256, 1229, 1166, 1017 $cm^{-1}$;
$^1H$ NMR (80 MHz, $CDCl_3$) δ (TMS): 8.20 (d, J= 8Hz, 1H, Ar), 7.8-7.2 (m, 7H, Ar), 6.90 (s, 2H, pyr), 6.30 (d, J= 8Hz, 1H, CHOPyr), 5.73 (d, J= 8Hz, 1H, CHOCO-), 3.64 (s, 3H, OMe), 3.42 (s, 3H, NMe), 1.29 (s, 3H, Me), 1.25 (s, 3H, Me).
Analysis Calcd. for $C_{28}H_{24}F_3N_3O_6$: C 60.54%; H 4.35%; N 7.56%. Found: C 60.29%; H4.39%; N 7.39%.

**B.- Title compound.**

Following the procedure described in example 3B, but starting from the product obtained in example 4A, the title compound of this example was obtained as a white solid (yield: 51%).
M.p.: 46-53 °C;
$[\alpha]D^{24}$ = +125.2 ° (0.5, $CHCl_3$);
IR (KBr) ν: 3600-3000, 2930, 2870, 2230, 1691, 1657, 1574, 1535, 1429, 1291, 1260, 1035, 840 $cm^{-1}$;
$^1H$ NMR (80 MHz, $CDCl_3$) δ (TMS): 8.18 (d, J= 8Hz, 1H, Ar), 7.76 (m, 2H, Ar), 7.07 (s, 2H, pyr), 6.18 (d, J= 8Hz, 1H, CHOPyr), 4.05 (d, J= 8Hz, 1H, CHOH), 3.67 (s, 3H, NMe), 3.24 (m, 1H, OH), 1.41 (s, 3H, Me), 1.27 (s, 3H, Me).
Analysis Calcd. for $C_{18}H_{17}N_3O_4$. 0.5 $CHCl_3$: C 55.89%; H 4.58%; N 10.25%. Found: C 55.67%; H4.39%; N 10.53%.

**EXAMPLE 5**

***Trans*-2,2-dimethyl-3-hydroxy-4-[6-hydroxy-1-(2-propenyl)-3-pyridazinyloxy]-1-oxo-1,2,3,4-tetrahydronaphthalen-6-carbonitrile**

Following the procedure described in example 2, but using allyl bromide instead of methyl iodide, the title compound of this example was obtained as a white solid (yield: 76 %).
M.p.: 156-157 °C;
IR (KBr) ν: 3600-3200, 2937, 1671, 1648, 1566, 1534, 1285, 1253, 1161, 1148, 1078, 843, 773 $cm^{-1}$;
$^1H$ NMR (80 MHz, $CDCl_3$) δ (TMS): 8.18 (d, J= 8Hz, 1H, Ar), 7.73 (m, 2H, Ar), 7.07 (s, 2H, pyr), 6.15 (d, J= 8.8Hz, 1H, CHO), 5.9 (m, 1H, CH=), 5.35 (s, 1H, CH=), 5.18 (m, 1H, CH=), 4.64 (d, J= 5.6Hz, 2H, $CH_2N$), 4.06 (d, J= 8.8Hz, 1H, CHOH), 2.0 (broad s., 1H, OH) 1.40 (s, 3H, $CH_3$), 1.25 (s, 3H, $CH_3$).
Analysis Calcd. for $C_{20}H_{19}N_3O_4$. 0.25 $H_2O$: C 64.95%; H 5.28%; N 11.37%. Found: C 64.81%; H 5.47%; N 11.92%.

**EXAMPLE 6**

***Trans*-3-acetoxy-4-(6-acetoxy-3-pyridazinyloxy)-2,2-dimethyl-1-oxo-1,2,3,4-tetrahydronaphthalen-6-carbonitrile**

To a solution of 0.200 g (0.6 mmol) of the product obtained in example 1 in 2.4 mL of pyridine, were added 1.2 mL of acetic anhydride and the mixture was stirred at room temperature for 24 h. After removal of the solvent, the residue was dissolved in $CH_2Cl_2$, washed with $NaHCO_3$ and $H_2O$ and dried over $MgSO_4$. The solvent was removed, affording 0.400 g of a crude that was cromatographed on silica gel eluting with hexane-ethyl acetate mixtures of increasing polarity. 0.200 g of the title compound of this example were obtained as a white solid (yield: 81%).
IR (KBr) $\nu$: 3600-3200, 2972, 2230, 1767, 1743, 1677, 1594, 1278, 1222, 1186 $cm^{-1}$;
$^1H$ NMR (80 MHz, $CDCl_3$) $\delta$ (TMS): 8.20 (d, J = 8Hz, 1H, Ar), 7.78 (m, 2H, Ar), 7.07 (s, 2H, pyr), 6.34 (d, J = 8Hz, 1H, CHO), 5.51 (d, J = 8Hz, 1H, CHOAc), 2.32 (s, 3H, $CH_3$), 2.03 (s, 3H, $CH_3$), 1.30 (s, 3H, $CH_3$), 1.27 (s, 3H, $CH_3$).

## EXAMPLE 7

### 1,2-dihydro-2,2-dimethyl-4-(6-hydroxy-3-pyridazinyloxy)-1-oxonaphthalen-6-carbonitrile

To a solution of 0.200 g (0.49 mmol) of the product obtained in example 6 in 3 mL of toluene, were added 0.09 mL (0.6 mmol) of 1,8-diazabicyclo(5.4.0)undec-7-ene (DBU) and the mixture was stirred at reflux under argon atmosphere overnight. The solvent was removed and the residue was dissolved in ethyl acetate. The solution was washed with $H_2O$ and dried over $MgSO_4$. The solvent was removed, affording 0.100 g of a crude that was cromatographed on silica gel eluting with hexane-ethyl acetate mixtures of increasing polarity. 0.070 g of the title compound of this example were obtained as a white solid (yield: 47%).
M.p.: 225-227 °C;
IR (KBr) $\nu$: 3600-3200, 2937, 1671, 1648, 1566, 1534, 1285, 1253, 1161, 1148, 1078, 843, 773 $cm^{-1}$;
$^1H$ NMR (80 MHz, $CDCl_3$) $\delta$ (TMS): 8.16 (d, J = 8Hz, 1H, Ar), 7.69 (m, 2H, Ar), 7.29 (d, J = 9.6Hz, 1H, pyr), 7.26 (s, 1H, NH), 7.06 (d, J = 9.6Hz, 1H, pyr), 5.97 (s, 1H, CH), 1.38 (s, 6H, $2CH_3$).
Analysis Calcd. for $C_{17}H_{13}N_3O_3$: C 66.44%; H 4.26%; N 13.67%. Found: C 66.33%; H 4.65%; N 13.49%.

## EXAMPLE 8

### *Trans*-6-bromo-2,2-dimethyl-3-hydroxy-4-(6-hydroxy-3-pyridazinyloxy)-1,2,3,4-tetrahydronaphthalen-1-one

Following the procedure described in example 1, but starting from 6-bromo-2,2-dimethyl-3,4-epoxy-1,2,3,4-tetrahydronaftalen-1-one (EP 489300), the title compound of this example was obtained as a white solid (yield: 21%).
M.p.: 247-250 °C;
IR (KBr) $\nu$: 3600-3200, 2929, 1683, 1664, 1648, 1591, 1428, 1284, 1052, 983 $cm^{-1}$;
$^1H$ NMR (80 MHz, $CDCl_3$-$CD_3OD$) $\delta$ (TMS): 7.92 (d, J = 8Hz, 1H, Ar), 7.60 (m, 2H, Ar), 7.25 (d, J = 9.6Hz, 1H, pyr), 7.01 (d, J = 9.6Hz, 1H, pyr), 6.17 (d, J = 8Hz, 1H, CHO), 4.25 (broad s, 2H, OH + NH + $H_2O$), 3.97 (d, J = 8Hz, 1H, CHOH), 1.34 (s, 3H, $CH_3$), 1.22 (s, 3H, $CH_3$).
Analysis Calcd. for $C_{16}H_{15}BrN_2O_4.0.25H_2O$: C 50.06%; H 4.04%; N 7.30%. Found: C 49.76%; H 4.06%; N 7.09%.

## EXAMPLE 9

### *Trans*-7-bromo-2,2-dimethyl-3-hydroxy-4-(2-pyridyloxy)-1,2,3,4-tetrahydronaphthalen-1-one

Following the procedure described in example 1, but starting from the product obtained in preparation 3 and using 2-hydroxypyridine instead of 3,6-dihydroxypyridazine, the title compound of this example was obtained as a white solid (yield: 15%).
M.p.: 142-144 °C;
IR (KBr) $\nu$: 3600-3200, 3063, 2963, 2927, 1674, 1588, 1566, 1464, 1427, 1397, 1267, 1247, 1011, 780 $cm^{-1}$;
$^1H$ NMR (80 MHz, $CDCl_3$) $\delta$ (TMS): 8.19 (m, 2H, Ar), 7.70 (m, 2H, Ar), 7.34 (m, 2H, Ar), 7.05 (m, 1H, Ar), 6.25 (d, J = 8.8Hz, 1H, CHO), 4.8 (broad s., 1H, OH), 4.02 (d, J = 8.8Hz, 1H, CHOH), 1.41 (s, 3H, $CH_3$), 1.25 (s, 3H, $CH_3$).
Analysis Calcd. for $C_{17}H_{16}BrNO_3$: C 56.37%; H 4.45%; N 3.87%. Found: C 56.79%; H 4.52%; N 3.84%.

## EXAMPLE 10

### *Trans*-6-bromo-2,2-dimethyl-3-hydroxy-4-(2-pyridyloxy)-1,2,3,4-tetrahydronaphthalen-1-one

Following the procedure described in example 1, but starting from 6-bromo-2,2-dimethyl-3,4-epoxy-1,2,3,4-tetrahydronaphthalen-1-one (EP 489300) and using 2-hydroxypyridine instead of 3,6-dihydroxypyridazine, the title compound of this example was obtained as a white solid (yield: 8%).
M.p.: 147-150 ° C;
IR (KBr) $\nu$: 3600-3200, 2967, 2929, 1683, 1580, 1462, 1424, 1281, 1263, 1072 $cm^{-1}$;
$^1H$ NMR (80 MHz, $CDCl_3$) $\delta$ (TMS): 8.3-7.4 (m, 5H, Ar), 7.02 (m, 2H, Ar), 6.28 (d, J = 9.6Hz, 1H, CHO), 4.02 (d, J = 9.6Hz, 1H, CHOH), 1.8 (broad s., 1H, OH), 1.41 (s, 3H, $CH_3$), 1.25 (s, 3H, $CH_3$).
Analysis Calcd. for $C_{17}H_{16}BrNO_3$: C 56.37%; H 4.45%; N 3.87%. Found: C 56.28%; H 4.66%; N 3.88%.

## EXAMPLE 11

### *Trans*-2,2-dimethyl-3-hydroxy-1-oxo-4-(2-pyridyloxy)-1,2,3,4-tetrahydronaphthalen-6-carbonitrile

Following the procedure described in example 1, but using 2-hydroxypyridine instead of 3,6-dihydroxypyridazine, the title compound of this example was obtained as a white solid (yield: 9%).
M.p.: 43-51 ° C;
IR (KBr) $\nu$: 3600-3200, 2967, 2929, 2229, 1691, 1590, 1463, 1426, 1267, 1245, 1001 $cm^{-1}$;
$^1H$ NMR (80 MHz, $CDCl_3$) $\delta$ (TMS): 8.18 (m, 2H, Ar), 7.70 (m, 3H, Ar), 7.09 (m, 2H, Ar), 6.32 (d, J = 9.2Hz, 1H, CHO), 4.06 (d, J = 8.8Hz, 1H, CHOH), 1.44 (s, 3H, $CH_3$), 1.34 (s, 1H, OH), 1.28 (s, 3H, $CH_3$).
Analysis Calcd. for $C_{17}H_{16}BrNO_3.0.25H_2O$: C 69.12%; H 5.28%; N 8.96%. Found: C69.41%; H 5.33%; N 8.57%.

## EXAMPLE 12

### *Trans*-2,2-dimethyl-3-hydroxy-1-oxo-4-(3-oxo-1-cyclopent-1-enyloxy)-1,2,3,4-tetrahydronaphthalen-6-carbonitrile.

To a solution of 0.38 g (3.9 mmol) of 1,3-cyclopentanedione in 37 mL of anhydrous tetrahydrofuran, were added under argon atmosphere 0.166 g (3.5 mmol) of NaH (55%). After 30 min, 0.70 g (3.5 mmol) of copper(I)bromide methylsulfide complex were added, followed by a solution of 0.5 g (2.3 mmol) of 2,2-dimethyl-3,4-epoxy-1-oxo-1,2,3,4-tetrahydronaftalen-6-carbonitrile (EP 489300) in 6 mL of anydrous tetrahydrofuran. The mixture was stirred at room temperature for 15 days and the solvent was removed. The residue was partitioned among $CHCl_3$ and $H_2O$ and the aqueous phase was extracted with $CHCl_3$. The combined organic extracts were dried over $MgSO_4$ and the solvent was removed. The residue was purified by flash chromatography (hexaneethyl acetate) yielding the desired product as a white solid (0.030 g, 4%) together with starting epoxide (0.300 g, 60%).
M.p.: 181 ° C;
IR (KBr) $\nu$ 3600-3200, 2967, 2929, 1683, 1580, 1462, 1424, 1281, 1263 $cm^{-1}$;
$^1H$ NMR (80 MHz, $CDCl_3$) $\delta$ (TMS): 8.20 (d, J = 8Hz, 1H, Ar), 7.76 (m, 2H, Ar), 5.68 (s, 1H, CH = ), 5.42 (d, J = 8Hz, 1H, CHO), 4.08 (d, J = 8Hz, 1H, CHOH), 2.70 (m, 5H, $2CH_2$ + OH), 1.39 (s, 3H, $CH_3$), 1.27 (s, 3H, $CH_3$).
Analysis Calcd. for $C_{18}N_{17}NO_4.2.25H_2O$: C 61.45%; H 6.12%; N 3.98%. Found: C 61.20%; H 6.06%; N 3.36%.

## EXAMPLE 13

### *Trans*-4-acetylamino-2,2-dimethyl-3-hydroxy-1-oxo-1,2,3,4-tetrahydronaphthalen-6-carbonitrile

To a solution of 0.20 g (0.9 mmol) of *trans*-4-amino-2,2-dimethyl-3-hydroxy-1-oxo-1,2,3,4-tetrahydronaftalen-6-carbonitrile (EP 489300) in 9 mL of chloroform, were added 0.12 mL (0.9 mmol) of triethylamine and 0.06 mL (0.9 mmol) of acetyl chloride and the mixture was stirred at room temperature for 30 min. The organic phase was washed with $H_2O$ and dried over $MgSO_4$. The solvent was removed and the residue was chromatographed on silica gel (hexane-ethyl acetate), yielding 0.22 g of the title compound of this example as a white solid (yield: 81%).

M.p.: 171-175°C;
IR (film) ν: 3600-3200, 2969, 2927, 2230, 1685, 1649, 1529, 1298, 1283 $cm^{-1}$;
$^1H$ NMR (80 MHz, $CDCl_3$) δ (TMS): 8.14 (d, J= 8Hz, 1H, Ar), 7.69 (m, 2H, Ar), 6.02 (d, J= 8Hz, 1H, NH), 5.34 (t, J= 9.6Hz, 1H, CHN), 3.76 (d, J= 9.6Hz, 1H, CHO), 2.22 (s, 3H, $CH_3$), 2.0 (broad signal, 1H, OH), 1.36 (s, 3H, $CH_3$), 1.17 (s, 3H, $CH_3$).
Analysis Calcd. for $C_{15}H_{16}N_2O_3$: C 59.31%; H 6.42%; N 9.23%. Found: C 59.23%; H 6.49%; N 8.55%.

**EXAMPLE 14**

***Trans*-2,2-dimethyl-3-hydroxy-1-oxo-4-(2-pyridylcarbonylamino)-1,2,3,4-tetrahydronaphthalen-6-carbonitrile**

To a solution of 0.20 g (0.9 mmol) of *trans*-4-amino-2,2-dimethyl-3-hydroxy-1-oxo-1,2,3,4-tetrahydronaftalen-6-carbonitrile (EP 489300) in 2.5 mL of anhydrous dimethylformamide, were added 0.176 g (0.9 mmol) of dicyclohexylcarbodiimide, 0.107g (0.9 mmol) of 2-picolinic acid and 0.116 g (0.9 mmol) of 1-hydroxybenzotriazol, and the mixture was stirred at room temperature for 18 h. The suspension was poured into ethyl acetate and filtered. The filtrate was washed with $NaHCO_3$, $H_2O$ and NaCl and dried over $MgSO_4$. The solvent was removed and the residue was chromatographed on silica gel (hexane-ethyl acetate), yielding 0.24 g of the title compound of this example as a white solid (yield: 82%).
M.p.: 207-208°C;
IR (film) ν: 3600-3200, 2964, 2929, 2229, 1691, 1664, 1518, 1460, 1427, 1405, 1291, 1086, 1070, 940 $cm^{-1}$;
$^1H$ NMR (80 MHz, $CDCl_3$) δ (TMS): 8.53 (m, 2H, Ar), 8.19 (m, 2H, Ar), 8.1-7.6 (m, 4H, Ar +NH), 5.55 (t, J= 9.6Hz, 1H, CHN), 3.97 (d, J= 9.6Hz, 1H, CHO), 3.0 (broad signal, 1H, OH), 1.40 (s, 3H, $CH_3$), 1.25 (s, 3H, $CH_3$).
Analysis Calcd. for $C_{19}N_{17}N_3O_3$.0.25 $H_2O$: C 67.16%; H 5.15%; N 12.37%. Found: C 67.23%; H 5.36%; N 12.03%.

**EXAMPLE 15**

***Trans*-2,2-dimethyl-3-hydroxy-1-oxo-4-(3-pyridylcarbonylamino)-1,2,3,4-tetrahydronaphthalen-6-carbonitrile**

Following the procedure described in example 14, but using nicotinic acid instead of 2-picolinic acid, the title compound of this example was obtained as a white solid (yield: 70%).
M.p.: 155-163°C;
IR (film) ν: 3600-3200, 2969, 2928, 2229, 1685, 1642, 1591, 1529, 1299, 1282, 1088, 1068 $cm^{-1}$;
$^1H$ NMR (80 MHz, $CDCl_3$) δ (TMS): 9.09 (m, 1H, Ar), 8.70 (m, 1H, Ar), 8.38 (d, J= 8Hz, 1H, Ar), 8.16 (d, J= 8Hz, 1H, Ar), 7.8-7.3 (m, 4H, Ar +NH), 5.47 (d, J= 9.6Hz, 1H, CHN), 3.92 (d, J= 9.6Hz, 1H, CHO), 3.0 (broad signal, 1H, OH+$H_2O$), 1.39 (s, 3H, $CH_3$), 1.23 (s, 3H, $CH_3$).
Analysis Calcd. for $C_{19}H_{17}N_3O_3$.1$H_2O$: C 64.59%; H 5.38%; N 11.90%. Found: C 64.65%; H 5.08%; N 11.75%.

**EXAMPLE 16**

***Trans*-2,2-dimethyl-3-hydroxy-1-oxo-4-(4-pyridylcarbonylamino)-1,2,3,4-tetrahydronaphthalen-6-carbonitrile**

Following the procedure described in example 14, but using 4-pyridylcarboxilic acid instead of 2-picolinic acid, the title compound of this example was obtained as a white solid (yield: 69%).
M.p.: 223°C;
IR (film) ν: 3600-3200, 2974, 2231, 1691, 1665, 1529, 1404, 1279, 1070 $cm^{-1}$;
$^1H$ NMR (80 MHz, $CDCl_3$) δ (TMS): 8.79 (m, 3H, Ar), 8.3-7.6 (m, 5H, Ar+NH), 5.46 (t, J= 9.6Hz, 1H, CHN), 3.94 (d, J= 9.6Hz, 1H, CHO), 3.92 (broad signal, 1H, OH), 1.39 (s, 3H, $CH_3$), 1.23 (s, 3H, $CH_3$).
Analysis Calcd. for $C_{19}H_{17}N_3O_3$: C 68.05%; H 5.11%; N 12.53%. Found: C 67.82%; H 5.21%; N 12.01%.

**EXAMPLE 17**

***Trans*-2,2-dimethyl-4-(3-furylcarbonylamino)-3-hydroxy-1-oxo-1,2,3,4-tetrahydronaphthalen-6-**

**carbonitrile**

Following the procedure described in example 14, but using 3-furoic acid instead of 2-picolinic acid, the title compound of this example was obtained as a white solid (yield: 67%).
M.p.: 220-224 °C;
IR (film) ν: 3600-3200, 2964, 2929, 2229, 1691, 1664, 1518, 1460, 1427, 1405, 1291, 1086, 1070, 940 $cm^{-1}$;
$^1H$ NMR (80 MHz, $CDCl_3$) δ (TMS): 8.68 (m, 3H, Ar), 7.8-7.4 (m, 3H, Ar+NH), 6.87 (broad s, 1H, Fur), 5.39 (m, 1H, CHN), 3.99 (broad signal, 1H, OH), 3.87 (d, J = 9.6Hz, 1H, CHO), 1.37 (s, 3H, $CH_3$), 1.21 (s, 3H, $CH_3$).
Analysis Calcd. for $C_{18}H_{16}N_2O_4$: C 66.66%; H 4.97%; N 8.64%. Found: C 66.42%; H 4.83%; N 8.87%.

**EXAMPLE 18**

***Trans*-2,2-dimethyl-3-hydroxy-1-oxo-4-(phenylcarbonylamino)-1,2,3,4-tetrahydronaphthalen-6-carbonitrile**

Following the procedure described in example 14, but using benzoic acid instead of 2-picolinic acid, the title compound of this example was obtained as a white solid (yield: 55%).
M.p.: 230-234 °C;
IR (film) ν: 3600-3200, 2964, 2929, 2229, 1691, 1664, 1518, 1460, 1427, 1405, 1291, 1086, 1070, 940 $cm^{-1}$;
$^1H$ NMR (80 MHz, $CDCl_3$) δ (TMS): 8.4-7.3 (m, 9H, Ar+NH), 5.46 (m, 1H, CHN), 4.0 (broad signal, 1H, OH), 3.93 (d, J = 9.6Hz, 1H, CHO), 1.39 (s, 3H, $CH_3$), 1.23 (s, 3H, $CH_3$).
Analysis Calcd. for $C_{20}H_{18}N_2O_3.0.5H_2O$: C 70.18%; H 5.55%; N 8.19%. Found: C 70.17%; H 5.59%; N 8.41%.

**EXAMPLE 19**

***Trans*-2,2-dimethyl-3-hydroxy-4-(4-methoxyphenylcarbonylamino)-1-oxo-1,2,3,4-tetrahydronaphthalen-6-carbonitrile**

Following the procedure described in example 14, but using 4-methoxybenzoic acid instead of 2-picolinic acid, the title compound of this example was obtained as a white solid (yield: 54%).
M.p.: 205-208 °C;
IR (film) ν: 3600-3200, 2970, 2927, 1683, 1627, 1602, 1496, 1283, 1256, 1187 $cm^{-1}$;
$^1H$ NMR (80 MHz, $CDCl_3$) δ (TMS): 8.2-7.5 (m, 5H, Ar), 6.98 (d, J = 8Hz, 2H, Ar), 5.41 (m, 1H, CHN), 3.90 (d, J = 9.6Hz, 1H, CHO), 3.89 (s, 3H, $OCH_3$), 3.80 (s, 2H, OH+NH), 1.38 (s, 3H, $CH_3$), 1.22 (s, 3H, $CH_3$).
Analysis Calcd. for $C_{21}H_{20}N_2O_4.0.25H_2O$: C 68.39%; H 5.56%; N 7.60%. Found: C 68.11%; H 5.65%; N 7.70%.

**EXAMPLE 20**

***Trans*-3-acetoxy-2,2-dimethyl-1-oxo-4-(2-pyridylcarbonylamino)-1,2,3,4-tetrahydronaphthalen-6-carbonitrile**

Following the procedure described in example 6, but starting from the product obtained in example 14, the title compound of this example was obtained as a white solid (yield: 92%).
M.p.: 193-197 °C;
IR (film) ν: 3321, 3057, 2982, 2925, 2229, 1734, 1690, 1658, 1513, 1245, 1233, 1029 $cm^{-1}$;
$^1H$ NMR (80 MHz, $CDCl_3$) δ (TMS): 8.7-7.4 (m, 8H, Ar + NH), 5.76 (t, J = 10.4Hz, 1H, CHN), 5.39 (d, J = 10.4Hz, 1H, CHO), 2.00 (s, 3H, $CH_3CO$), 1.33 (s, 6H, $2CH_3$).

**EXAMPLE 21**

***Trans*-1,2-dihydro-2,2-dimethyl-1-oxo-4-(2-pyridylcarbonylamino)naphthalen-6-carbonitrile**

Following the procedure described in example 7, but starting from the product obtained in example 20, the title compound of this example was obtained as a white solid (yield: 67%).
M.p.: 147.8C °C;

IR (film) ν: 3342, 3062, 2962, 2225, 1680, 1523, 1476, 1428, 1311, 1284, 1219 $cm^{-1}$;
$^1H$ NMR (80 MHz, $CDCl_3$) δ (TMS): 9.75 (m, 1H, NH), 8.71 (m, 1H, Ar), 8.3-7.4 (m, 6H, Ar), 6.83 (s, 1H, CH=), 1.42 (s, 6H, $2CH_3$).
Analysis Calcd. for $C_{19}H_{15}N_3O_2$: C 71.91%; H 4.76%; N 13.24%. Found: C 71.57%; H 4.76%; N 13.09%.

## EXAMPLE 22

### *Trans*-4-(N-cyanoacetimidoylamino)-2,2-dimethyl-3-hydroxy-1-oxo-1,2,3,4-tetrahydronaphthalen-6-carbonitrile

To 0.54 g (4.8 mmol) of ethyl N-cyanoacetimidate, 0.200 g (0.9 mmol) of *trans*-4-amino-2,2-dimethyl-3-hydroxy-1-oxo-1,2,3,4-tetrahydronaftalen-6-carbonitrile (EP 489300) were added, and the mixture was stirred at 100 °C overnight. The solvent was removed, affording a crude that was chromatographed on silica gel (hexane-ethyl acetate). 0.10 g of the title compound of this example were obtained as a white solid (yield: 40%).
M.p.: 207-209 °C;
IR (film) ν: 3600-3200, 3112, 2978, 2228, 2184, 1679, 1573, 1407, 1300, 1057 $cm^{-1}$;
$^1H$ NMR (80 MHz, $CDCl_3$) δ (TMS): 8.84 (m, 1H, NH), 8.15 (d, J= 8Hz, 1H, Ar), 7.71 (m, 2H, Ar), 5.39 (m, 1H, CHN), 4.17 (s, 1H, OH), 3.80 (d, J= 9.6Hz, 1H, CHO), 2.49 (s, 3H, $CH_3$), 1.36 (s, 3H, $CH_3$), 1.19 (s, 3H, $CH_3$).
Analysis Calcd. for $C_{16}H_{16}N_4O_2$.0.25 $H_2O$: C 63.89%; H 5.49%; N 18.63%. Found: C 63.75%; H 5.52%; N 17.71%.

## EXAMPLE 23

### *Trans*-4-(6-chloropyrimidin-4-yl)-2,2-dimethyl-3-hydroxy-1-oxo-1,2,3,4-tetrahydronaphthalen-6-carbonitrile

To a solution of 0.25 g (1.1 mmol) of *trans*-4-amino-2,2-dimethyl-3-hydroxy-1-oxo-1,2,3,4-tetrahydronaftalen-6-carbonitrile (EP 489300) in 4 mL of ethanol, were added 0.15 mL (1.1 mmol) of triethylamine and 0.177 g (1.2 mmol) of 4,6-dichloropyrimidine, and the mixture was stirred at room temperature for 24 h. The solvent was removed and the residue was partitioned between $CHCl_3$ and $H_2O$. The aqueous phase was extracted with $CHCl_3$ and the combined organic phases were dried over $MgSO_4$. The solvent was removed, affording a crude that was chromatographed on silica gel (hexane-ethyl acetate). 0.035 g of the title compound of this example were obtained as a white solid (yield: 9%).
M.p.: 167-169 °C;
IR (film) ν: 3600-3200, 2965, 2929, 2230, 1690, 1577, 1494, 1094, 1048 $cm^{-1}$;
$^1H$ NMR (80 MHz, $CD_3OD$) δ (TMS): 8.34 (s, 1H, pyr), 8.15 (d, J= 8Hz, 1H, Ar), 7.70 (m, 2H, Ar), 6.70 (s, 1H, pyr), 5.53 (m, 1H, CHN), 3.95 (s, 2H, OH+NH+ Solv), 3.83 (d, J= 9.6Hz, 1H, CHO), 1.38 (s, 3H, $CH_3$), 1.24 (s, 3H, $CH_3$).
Analysis Calcd. for $C_{17}H_{15}ClN_4O_2$: C 59.57%; H 4.41%; N 16.34%. Found: C 59.65%; H 4.86%; N 15.26%.

## EXAMPLE 24

### *Trans*-4-[(4-amino-1,2-dioxo-3-cyclobuten-3-yl)amino]-2,2-dimethyl-3-hydroxy-1-oxo-1,2,3,4-tetrahydronaphthalen-6-carbonitrile

To a solution of 0.25 g (1.1 mmol) of *trans*-4-amino-2,2-dimethyl-3-hydroxy-1-oxo-1,2,3,4-tetrahydronaftalen-6-carbonitrile (EP 489300) in 8 mL of ethanol, were added 0.213 g (1.25 mmol) of 3,4-diethoxycyclobuten-1,2-dione and the mixture was stirred under nitrogen atmosphere for 18 h. 3 mL of a saturated solution of $NH_3$ in ethanol were added and the resulting mixture was stirred for 4 h more. The precipitate formed was filtered and dried, yielding 0.192 g of the title compound of this example as a white solid (yield: 54%).
M.p.: 291 °C;
IR (film) ν: 3600-3000, 2969, 2927, 2232, 1799, 1690, 1653, 1565, 1522, 1406 $cm^{-1}$;
$^1H$ NMR (80 MHz, DMSO) δ (TMS): 8.01 (m, 4H, Ar+$NH_2$+OH), 7.50 (m, 2H, Ar), 5.84 (m, 1H, NH), 5.16 (m, 1H, CHN), 3.83 (m, 1H, CHO), 1.23 (s, 3H, $CH_3$), 1.07 (s, 3H, $CH_3$).
Analysis Calcd. for $C_{17}H_{15}N_3O_4$.0.25$H_2O$: C 61.91%; H 4.70%; N 12.75%. Found: C 61.64%; H 4.61%; N

12.79%.

**Claims**

1. A compound of formula **I**:

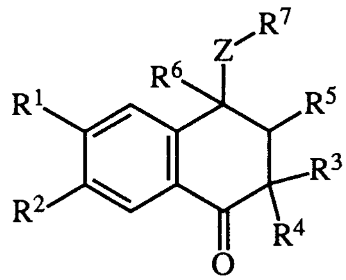

**I**

wherein:

**$R^1$** and **$R^2$** represent hydrogen, $C_{1-4}$ alkyl, hydroxyl, $C_{1-4}$ alkoxy, formyl, $C_{1-4}$ alkylcarbonyl, $C_{1-4}$ alkylthiocarbonyl, carboxyl, $C_{1-4}$ alkoxycarbonyl, $C_{1-4}$ alkoxythiocarbonyl, $C_{1-4}$ alkylcarbonyloxy, $C_{1-4}$ alkylthiocarbonyloxy, hydroxy($C_{1-4}$) alkyl, mercapto-($C_{1-4}$) alkyl, perfluoro($C_{1-4}$)alkyl, nitro, amino, cyano, halogen, trifluoromethoxy, ethynyl, trimethylsilylethynyl, $C_{1-4}$ alkylsulfinyl, arylsulfinyl, $C_{1-4}$ alkylsulfonyl, arylsulfonyl, $C_{1-4}$ alkoxysulfinyl, $C_{1-4}$ alkoxysulfonyl, $C_{1-4}$ alkylcarbonylamino, $C_{1-4}$ alkoxycarbonylamino, aminosulfinyl, aminosulfonyl, aminocarbonyl, aminothiocarbonyl, $C_{1-4}$ alkylsulfinylamino, $C_{1-4}$ alkylsulfonylamino, $C_{1-4}$ alkoxysulfinylamino, $C_{1-4}$ alkoxysulfonylamino, ($C_{1-4}$ alkyl)-carbonyl($C_{1-4}$ alkyl), nitro-($C_{1-4}$ alkyl), cyano($C_{1-4}$ alkyl), ($C_{1-4}$ alkyl)C(=NOH), ($C_{1-4}$ alkyl)C(=$NNH_2$) or ($C_{1-4}$ alkoxy)C(=NH), being the above amino groups optionally substituted by one or two $C_{1-4}$ alkyl groups;

**$R^3$** is hydrogen or $C_{1-4}$ alkyl; **$R^4$** is $C_{1-4}$ alkyl, or $R^3$ and $R^4$ together form a $C_{2-5}$ polymethylene chain;

either **$R^5$** is hydroxyl, acetoxy or formyloxy and **$R^6$** is hydrogen; or $R^5$ and $R^6$ together form a bond;

Z represents O or $NR^8$ such that

a) when Z represents O, then **$R^7$** is a group $R^9$, wherein $R^9$ is $C_{3-6}$ cycloalkyl, $C_{3-6}$ cycloalkenyl, phenyl or heteroaryl, all of them being optionally substituted by one or two halogen atoms and/or one or two $C_{1-6}$ alkyl, $C_{2-4}$ alkenylmethyl, $C_{2-4}$ alkynylmethyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups;

b) when Z represents $NR^8$, then **$R^7$** represents a radical $R^9$ or C(=X)$R^{10}$, where:

$R^8$ means hydrogen, $C_{1-6}$ alkyl, $C_{1-6}$ alkoxy, aryloxy, arylmethyloxy, $C_{1-6}$ alkylcarbonyloxy, arylcarbonyloxy, $C_{1-6}$ alkylcarbonylamino or arylcarbonylamino;

$R^9$ has the previously defined meaning;

X represents O, S or NCN; and

$R^{10}$ is hydrogen, $C_{1-6}$ alkyl (which may be optionally substituted by an hydroxy, $C_{1-6}$ alkoxy or $C_{1-6}$ alkoxycarbonyl group), $C_{2-6}$ alkenyl, $C_{3-6}$ cycloalkyl, $C_{3-6}$ cycloalkenyl, phenyl or heteroaryl, these last two radicals being optionally substituted by a group chosen from halogen, $C_{1-6}$ alkyl, $C_{1-6}$alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups;

and the salts thereof.

2. A compound according to claim 1 of formula **Ia**

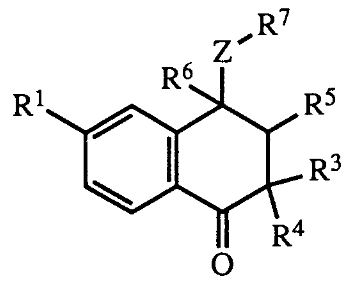

**Ia**

wherein $R^1$, $R^3$, $R^4$, $R^5$, $R^6$, $R^7$ and Z have the previously defined meaning

**3.** A compound according to claims 1 and 2 of formula **Ib**

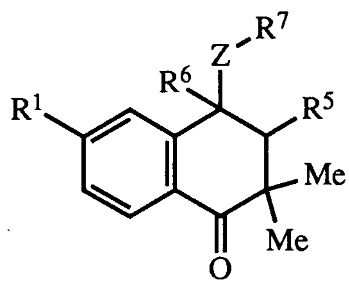

**Ib**

wherein $R^1$, $R^5$, $R^6$, $R^7$ and Z have the previously defined meaning.

**4.** A compound according to anyone claims 1-3 of formula **Ic**

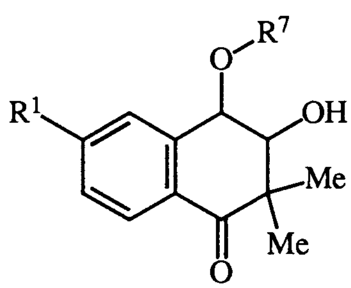

**Ic**

wherein $R^1$ and $R^7$ have the previously defined meaning.

**5.** A compound according to anyone claims 1-3 of formula **Id**

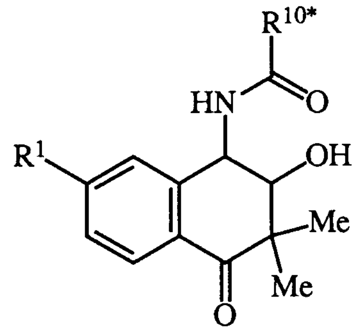

**Id**

wherein $R^1$ has the previously defined meaning, and $R^{10*}$ means phenyl or heteroaryl, both optionally substituted by a group chosen from halogen, $C_{1-6}$ alkyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups.

**6.**

a) *Trans* 2,2-dimethyl-3-hydroxy-4-(6-hydroxy-3-pyridazinyloxy)-1-oxo-1,2,3,4-tetrahydronaphthalen-6-carbonitrile and the salts thereof.

b) *Rac-Trans*-2,2-dimethyl-3-hydroxy-4-(6-hydroxy-1-methyl-3-pyridazinyloxy)-1-oxo-1,2,3,4-tetrahydronaphthalen-6-carbonitrile and the salts thereof.

c) *(-)-Trans*-2,2-dimethyl-3-hydroxy-4-(6-hydroxy-1-methyl-3-pyridazinyloxy)-1-oxo-1,2,3,4-tetrahydronaphthalen-6-carbonitrile and the salts thereof.

d) *Trans*-2,2-dimethyl-3-hydroxy-4-(4-methoxyphenylcarbonylamino)-1-oxo-1,2,3,4-tetrahydronaphthalen-6-carbonitrile and the salts thereof.

**7.** A process for preparing a compound according to Claim 1 which comprises:

(a) when in a compound of general formula **I**, $R^5$ is OH, $R^6$ is H and Z represents O, reacting a compound of general formula **V**, wherein $R^{1'}$ and $R^{2'}$ are $R^1$ or $R^2$ as defined above or a group or atom convertible thereto, and $R^3$ and $R^4$ have the previously defined meaning,

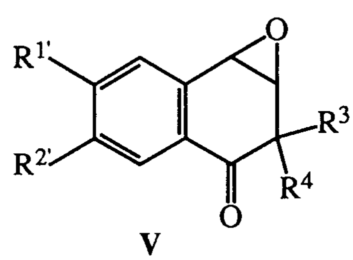

**V**

with a compound of general formula $R^9$-OH (**IX**, wherein $R^9$ has the previously defined meaning) in the presence of a base such as pyridine in a suitable solvent such as a polar solvent, for example ethanol, and optionally, transforming said $R^9$ into other groups $R^9$ by performing standard substitution reactions such as alkylation with an alkyl halide;

(b) when in a compound of general formula **I**, $R^5$ is OH, $R^6$ is H and Z represents $NR^8$, reacting a compound of general formula **V** with a compound of general formula $HNR^8R^9$ (**X**, wherein $R^8$ and $R^9$ have the previously defined meaning) in the presence of a base such as pyridine in a suitable solvent such as a polar solvent, for example ethanol; or reacting **V** with a compound of general formula $H_2NR^8$ (**XI**, wherein $R^8$ has the previously defined meaning) in the same experimental conditions, to give a compound of general formula **VI**:

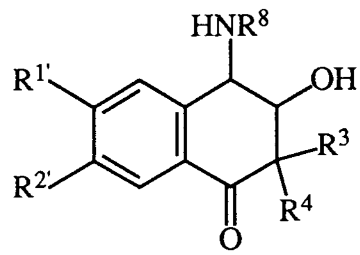

VI

wherein $R^{1'}$, $R^{2'}$, $R^3$, $R^4$ and $R^8$ have the previously defined meaning, and then reacting said compound **VI** with a compound of general formula Y-$R^9$ (**XII**, wherein $R^9$ has the previously defined meaning and Y represents a good leaving group such as an halogen atom or an alkoxy group) in the presence of a base such as triethylamine in a suitable solvent such as a polar solvent, for example ethanol; or reacting a compound of formula **VI** with a compound of general formula Y-C(=X)-$R^{10}$ - (**XIII**, wherein Y, X and $R^{10}$ have the previously defined meaning) in the presence of a base such as triethylamine in a suitable solvent such as compound **XIII** itself or chloroform, or alternatively, reacting **VI** with a compound of general formula HO-C(=X)-$R^{10}$ (**XIV**, wherein X and $R^{10}$ have the previously defined meaning) in the presence of a dehydrating agent such as diciclohexylcarbodiimide in a polar solvent such as dimethylformamide;

(c) in all cases wherein X is sulphur, said compounds may also be obtained by reacting a compound of formula **I** wherein X is oxygen with a thiation reagent such as Lawesson's reagent in a suitable solvent such as toluene;

(d) in all cases wherein $R^5$ is acetoxy and $R^6$ is hydrogen, reacting a compound of formula **I** wherein $R^5$ is hydroxyl and $R^6$ is hydrogen with acetic anhydride in the presence of a base such as pyridine;

(e) in all cases wherein $R^5$ is formyloxy and $R^6$ is hydrogen, reacting a compound of formula **I** wherein $R^5$ is hydroxyl and $R^6$ is hydrogen with formic acid in the presence of a base such as pyridine;

(f) in all cases wherein $R^5$ and $R^6$ together form a bond, reacting a compound of general formula **I** wherein $R^5$ is OH and $R^6$ is hydrogen with a base such as sodium hydride or sodium hydroxyde in a suitable solvent such as toluene or dioxane; or reacting a compound of general formula **I** wherein $R^5$ is acetoxy and $R^6$ is hydrogen with a base such as 1,8-diazabicyclo(5.4.0)undec-7-ene (DBU) in an inert solvent such as toluene;

(g) optionally, interconverting the groups $R^1$, $R^2$, $R^{1'}$ and/or $R^{2'}$ in a compound of formula **I** or any intermediate of formula **II-VI** into other groups $R^1$ and/or $R^2$;

(h) and optionally, reacting a compound of formula **I** with an acid to give its corresponding acid addition salt.

**8.** A pharmaceutical composition comprising an effective amount of at least one compound of formula **I** or a pharmaceutically acceptable salt thereof in admixture with a pharmaceutically acceptable excipient.

**9.** The use of at least one compound of formula **I** as defined in claim 1 for the manufacture of a medicament for the treatment and/or prevention of the diseases related with the regulation of the smooth muscle contraction at the cardiovascular, respiratory and cerebrovascular systems, and at the gastrointestinal, urinary and uterus tracts, and particularly for the treatment and/or prevention of hypertension and asthma in mammals, including man.

**Claims for the following Contracting State : GR**

**1.** A process forpreparing a compound of formula **I**:

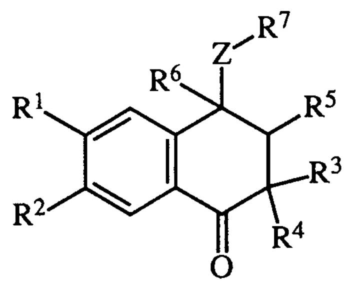

I

wherein:

**$R^1$** and **$R^2$** represent hydrogen, $C_{1-4}$ alkyl, hydroxyl, $C_{1-4}$ alkoxy, formyl, $C_{1-4}$ alkylcarbonyl, $C_{1-4}$ alkylthiocarbonyl, carboxyl, $C_{1-4}$ alkoxycarbonyl, $C_{1-4}$ alkoxythiocarbonyl, $C_{1-4}$ alkylcarbonyloxy, $C_{1-4}$ alkylthiocarbonyloxy, hydroxy($C_{1-4}$) alkyl, mercapto-($C_{1-4}$) alkyl, perfluoro($C_{1-4}$)alkyl, nitro, amino, cyano, halogen, trifluoromethoxy, ethynyl, trimethylsilylethynyl, $C_{1-4}$ alkylsulfinyl, arylsulfinyl, $C_{1-4}$ alkylsulfonyl, arylsulfonyl, $C_{1-4}$ alkoxysulfinyl, $C_{1-4}$ alkoxysulfonyl, $C_{1-4}$ alkylcarbonylamino, $C_{1-4}$ alkoxycarbonylamino, aminosulfinyl, aminosulfonyl, aminocarbonyl, aminothiocarbonyl, $C_{1-4}$ alkylsulfinylamino, $C_{1-4}$ alkylsulfonylamino, $C_{1-4}$ alkoxysulfinylamino, $C_{1-4}$ alkoxysulfonylamino, ($C_{1-4}$ alkyl)-carbonyl($C_{1-4}$ alkyl), nitro-($C_{1-4}$ alkyl), cyano($C_{1-4}$ alkyl), ($C_{1-4}$ alkyl)C(=NOH), ($C_{1-4}$ alkyl)C-(=$NNH_2$) or ($C_{1-4}$ alkoxy)C(=NH), being the above amino groups optionally substituted by one or two $C_{1-4}$ alkyl groups;

**$R^3$** is hydrogen or $C_{1-4}$ alkyl; **$R^4$** is $C_{1-4}$ alkyl, or $R^3$ and $R^4$ together form a $C_{2-5}$ polymethylene chain;

either **$R^5$** is hydroxyl, acetoxy or formyloxy and **$R^6$** is hydrogen; or $R^5$ and $R^6$ together form a bond;

**Z** represents O or $NR^8$ such that

a) when Z represents O, then **$R^7$** is a group $R^9$, wherein $R^9$ is $C_{3-6}$ cycloalkyl, $C_{3-6}$ cycloalkenyl, phenyl or heteroaryl, all of them being optionally substituted by one or two halogen atoms and/or one or two $C_{1-6}$ alkyl, $C_{2-4}$ alkenylmethyl, $C_{2-4}$ alkynylmethyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups;

b) when Z represents $NR^8$, then **$R^7$** represents a radical $R^9$ or C(=X)$R^{10}$, where:

$R^8$ means hydrogen, $C_{1-6}$ alkyl, $C_{1-6}$ alkoxy, aryloxy, arylmethyloxy, $C_{1-6}$ alkylcarbonyloxy, arylcarbonyloxy, $C_{1-6}$ alkylcarbonylamino or arylcarbonylamino;

$R^9$ has the previously defined meaning;

X represents O, S or NCN; and

$R^{10}$ is hydrogen, $C_{1-6}$ alkyl (which may be optionally substituted by an hydroxy, $C_{1-6}$ alkoxy or $C_{1-6}$ alkoxycarbonyl group), $C_{2-6}$ alkenyl, $C_{3-6}$ cycloalkyl, $C_{3-6}$ cycloalkenyl, phenyl or heteroaryl, these last two radicals being optionally substituted by a group chosen from halogen, $C_{1-6}$ alkyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups;

and the salts thereof,

which comprises:

(a) when in a compound of general formula **I**, $R^5$ is OH, $R^6$ is H and Z represents O, reacting a compound of general formula **V**, wherein $R^{1'}$ and $R^{2'}$ are $R^1$ or $R^2$ as defined above or a group or atom convertible thereto, and $R^3$ and $R^4$ have the previously defined meaning,

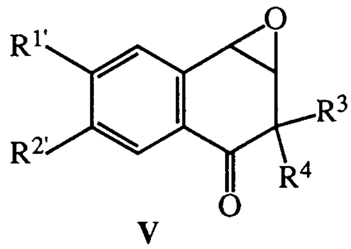

V

with a compound of general formula $R^7$-OH (**IX,** wherein $R^7$ has the previously defined meaning) in the presence of a base such as pyridine in a suitable solvent such as a polar solvent, for example ethanol, and optionally, transforming said $R^9$ into other groups $R^9$ by performing standard substitution reactions such as alkylation with an alkyl halide;

(b) when in a compound of general formula **I**, $R^5$ is OH, $R^6$ is H and Z represents $NR^8$, reacting a compound of general formula **V** with a compound of general formula $HNR^8R^9$ (**X**, wherein $R^8$ and $R^9$ have the previously defined meaning) in the presence of a base such as pyridine in a suitable solvent such as a polar solvent, for example ethanol; or reacting **V** with a compound of general formula $H_2NR^8$ (**XI**, wherein $R^8$ has the previously defined meaning) in the same experimental conditions, to give a compound of general formula **VI**:

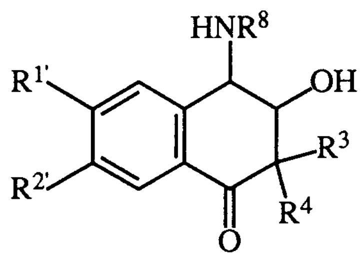

VI

wherein $R^{1'}$, $R^{2'}$, $R^3$, $R^4$ and $R^8$ have the previously defined meaning, and then reacting said compound **VI** with a compound of general formula Y-$R^9$ (**XII**, wherein $R^9$ has the previously defined meaning and Y represents a good leaving group such as an halogen atom or an alkoxy group) in the presence of a base such as triethylamine in a suitable solvent such as a polar solvent, for example ethanol; or reacting a compound of formula **VI** with a compound of general formula Y-C(=X)-$R^{10}$ - (**XIII**, wherein Y, X and $R^{10}$ have the previously defined meaning) in the presence of a base such as triethylamine in a suitable solvent such as compound **XIII** itself or chloroform, or alternatively, reacting **VI** with a compound of general formula HO-C(=X)-$R^{10}$ (**XIV**, wherein X and $R^{10}$ have the previously defined meaning) in the presence of a dehydrating agent such as diciclohexylcarbodiimide in a polar solvent such as dimethylformamide;

(c) in all cases wherein X is sulphur, said compounds may also be obtained by reacting a compound of formula **I** wherein X is oxygen with a thiation reagent such as Lawesson's reagent in a suitable solvent such as toluene;

(d) in all cases wherein $R^5$ is acetoxy and $R^6$ is hydrogen, reacting a compound of formula **I** wherein $R^5$ is hydroxyl and $R^6$ is hydrogen with acetic anhydride in the presence of a base such as pyridine;

(e) in all cases wherein $R^5$ is formyloxy and $R^6$ is hydrogen, reacting a compound of formula **I** wherein $R^5$ is hydroxyl and $R^6$ is hydrogen with formic acid in the presence of a base such as pyridine;

(f) in all cases wherein $R^5$ and $R^6$ together form a bond, reacting a compound of general formula **I** wherein $R^5$ is OH and $R^6$ is hydrogen with a base such as sodium hydride or sodium hydroxyde in a suitable solvent such as toluene or dioxane; or reacting a compound of general formula **I** wherein $R^5$ is acetoxy and $R^6$ is hydrogen with a base such as 1,8-diazabicyclo(5.4.0)undec-7-ene (DBU) in an inert solvent such as toluene;

(g) optionally, interconverting the groups $R^1$, $R^2$, $R^{1'}$ and/or $R^{2'}$ in a compound of formula **I** or any

intermediate of formula **II-VI** into other groups $R^1$ and/or $R^2$;
(h) and optionally, reacting a compound of formula **I** with an acid to give its corresponding acid addition salt.

2. A process according to Claim 1 in which the reagents and reaction conditions are so chosen as to prepare a compound of formula **Ia**

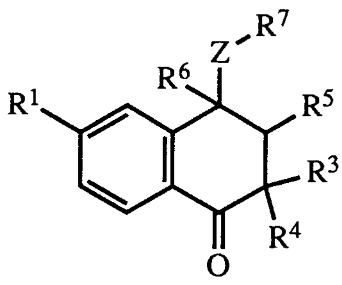

**Ia**

wherein $R^1$, $R^3$, $R^4$, $R^5$, $R^6$, $R^7$ and Z have the previously defined meaning.

3. A process according to Claim 1 in which the reagents and reaction conditions are so chosen as to prepare a compound of formula **Ib**

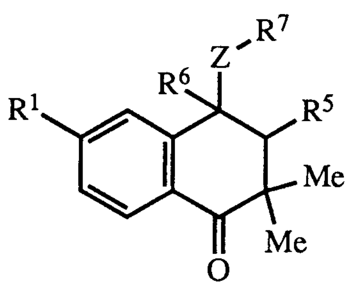

**Ib**

wherein $R^1$, $R^5$, $R^6$, $R^7$ and Z have the previously defined meaning.

4. A process according to Claim 1 in which the reagents and reaction conditions are so chosen as to prepare a compound of formula **Ic**

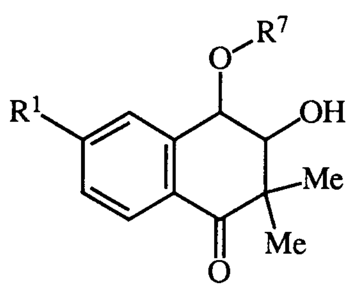

**Ic**

wherein $R^1$ and $R^7$ have the previously defined meaning.

5. A process according to Claim 1 in which the reagents and reaction conditions are so chosen as to

prepare a compound of formula **Id**

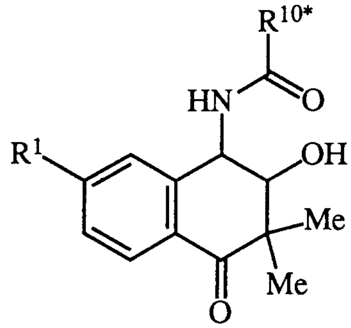

**Id**

wherein $R^1$ has the previously defined meaning, and $R^{10*}$ means phenyl or heteroaryl, both optionally substituted by a group chosen from halogen, $C_{1-6}$ alkyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups.

**6.**

a) A process according to Claim 1 in which the reagents and reaction conditions are so chosen as to prepare *trans* -2,2-dimethyl-3-hydroxy-4-(6-hydroxy-3-pyridazinyloxy)-1-oxo-1,2,3,4-tetrahydronaphthalen-6-carbonitrile and the salts thereof.

b) A process according to Claim 1 in which the reagents and reaction conditions are so chosen as to prepare *rac-trans*-2,2-dimethyl-3-hydroxy-4-(6-hydroxy-1-methyl-3-pyridazinyloxy)-1-oxo-1,2,3,4-tetrahydronaphthalen-6-carbonitrile and the salts thereof.

c) A process according to Claim 1 in which the reagents and reaction conditions are so chosen as to prepare *(-)-trans*-2,2-dimethyl-3-hydroxy-4-(6-hydroxy-1-methyl-3-pyridazinyloxy)-1-oxo-1,2,3,4-tetrahydronaphthalen-6-carbonitrile and the salts thereof.

d) A process according to Claim 1 in which the reagents and reaction conditions are so chosen as to prepare *trans*-2,2-dimethyl-3-hydroxy-4-(4-methoxyphenylcarbonylamino)-1-oxo-1,2,3,4-tetrahydronaphthalen-6-carbonitrile and the salts thereof.

**7.** The use of at least one compound of formula **I** as defined in claim 1 for the manufacture of a medicament for the treatment and/or prevention of the diseases related with the regulation of the smooth muscle contraction at the cardiovascular, respiratory and cerebrovascular systems, and at the gastrointestinal, urinary and uterus tracts, and particularly for the treatment and/or prevention of hypertension and asthma in mammals, including man.

**Claims for the following Contracting State : ES**

**1.** A process for preparing a compound of formula **I**:

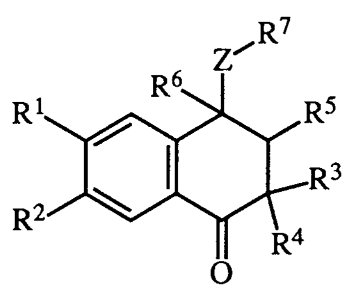

**I**

wherein:

**$R^1$** and **$R^2$** represent hydrogen, $C_{1-4}$ alkyl, hydroxyl, $C_{1-4}$ alkoxy, formyl, $C_{1-4}$ alkylcarbonyl, $C_{1-4}$ alkylthiocarbonyl, carboxyl, $C_{1-4}$ alkoxycarbonyl, $C_{1-4}$ alkoxythiocarbonyl, $C_{1-4}$ alkylcarbonyloxy, $C_{1-4}$ alkylthiocarbonyloxy, hydroxy($C_{1-4}$) alkyl, mercapto-($C_{1-4}$) alkyl, perfluoro($C_{1-4}$)alkyl, nitro, amino, cyano, halogen, trifluoromethoxy, ethynyl, trimethylsilylethynyl, $C_{1-4}$ alkylsulfinyl, arylsulfinyl, $C_{1-4}$ alkylsulfonyl, arylsulfonyl, $C_{1-4}$ alkoxysulfinyl, $C_{1-4}$ alkoxysulfonyl, $C_{1-4}$ alkylcarbonylamino, $C_{1-4}$ alkoxycarbonylamino, aminosulfinyl, aminosulfonyl, aminocarbonyl, aminothiocarbonyl, $C_{1-4}$ alkylsulfinylamino, $C_{1-4}$ alkylsulfonylamino, $C_{1-4}$ alkoxysulfinylamino, $C_{1-4}$ alkoxysulfonylamino, ($C_{1-4}$ alkyl)-carbonyl($C_{1-4}$ alkyl), nitro-($C_{1-4}$ alkyl), cyano($C_{1-4}$ alkyl), ($C_{1-4}$ alkyl)C(=NOH), ($C_{1-4}$ alkyl)C(=$NNH_2$) or ($C_{1-4}$ alkoxy)C(=NH), being the above amino groups optionally substituted by one or two $C_{1-4}$ alkyl groups;

**$R^3$** is hydrogen or $C_{1-4}$ alkyl; **$R^4$** is $C_{1-4}$ alkyl, or $R^3$ and $R^4$ together form a $C_{2-5}$ polymethylene chain;

either **$R^5$** is hydroxyl, acetoxy or formyloxy and **$R^6$** is hydrogen; or $R^5$ and $R^6$ together form a bond;

**Z** represents O or $NR^8$ such that

a) when Z represents O, then **$R^7$** is a group $R^9$, wherein $R^9$ is $C_{3-6}$ cycloalkyl, $C_{3-6}$ cycloalkenyl, phenyl or heteroaryl, all of them being optionally substituted by one or two halogen atoms and/or one or two $C_{1-6}$ alkyl, $C_{2-4}$ alkenylmethyl, $C_{2-4}$ alkynylmethyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups;

b) when Z represents $NR^8$, then **$R^7$** represents a radical $R^9$ or $C(=X)R^{10}$, where:

$R^8$ means hydrogen, $C_{1-6}$ alkyl, $C_{1-6}$ alkoxy, aryloxy, arylmethyloxy, $C_{1-6}$ alkylcarbonyloxy, arylcarbonyloxy, $C_{1-6}$ alkylcarbonylamino or arylcarbonylamino;

$R^9$ has the previously defined meaning;

X represents O, S or NCN; and

$R^{10}$ is hydrogen, $C_{1-6}$ alkyl (which may be optionally substituted by an hydroxy, $C_{1-6}$ alkoxy or $C_{1-6}$ alkoxycarbonyl group), $C_{2-6}$ alkenyl, $C_{3-6}$ cycloalkyl, $C_{3-6}$ cycloalkenyl, phenyl or heteroaryl, these last two radicals being optionally substituted by a group chosen from halogen, $C_{1-6}$ alkyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups;

and the salts thereof,

which comprises:

(a) when in a compound of general formula **I**, $R^5$ is OH, $R^6$ is H and Z represents O, reacting a compound of general formula **V**, wherein $R^{1'}$ and $R^{2'}$ are $R^1$ or $R^2$ as defined above or a group or atom convertible thereto, and $R^3$ and $R^4$ have the previously defined meaning,

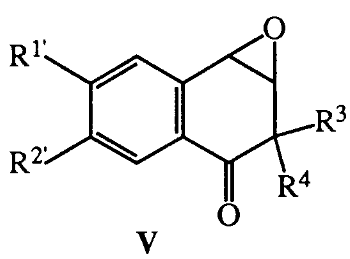

**V**

with a compound of general formula $R^7$-OH (**IX**, wherein $R^7$ has the previously defined meaning) in the presence of a base such as pyridine in a suitable solvent such as a polar solvent, for example ethanol, and optionally, transforming said $R^9$ into other groups $R^9$ by performing standard substitution reactions such as alkylation with an alkyl halide;

(b) when in a compound of general formula **I**, $R^5$ is OH, $R^6$ is H and Z represents $NR^8$, reacting a compound of general formula **V** with a compound of general formula $HNR^8R^9$ (**X**, wherein $R^8$ and $R^9$ have the previously defined meaning) in the presence of a base such as pyridine in a suitable solvent such as a polar solvent, for example ethanol; or reacting **V** with a compound of general formula $H_2NR^8$ (**XI**, wherein $R^8$ has the previously defined meaning) in the same experimental

conditions, to give a compound of general formula **VI**:

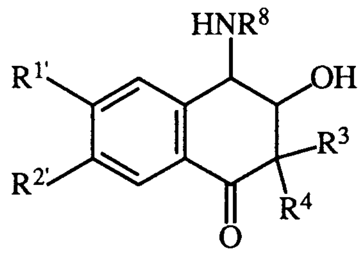

**VI**

wherein $R^{1'}$, $R^{2'}$, $R^3$, $R^4$ and $R^8$ have the previously defined meaning, and then reacting said compound **VI** with a compound of general formula Y-$R^9$ (**XII**, wherein $R^9$ has the previously defined meaning and Y represents a good leaving group such as an halogen atom or an alkoxy group) in the presence of a base such as triethylamine in a suitable solvent such as a polar solvent, for example ethanol; or reacting a compound of formula **VI** with a compound of general formula Y-C(=X)-$R^{10}$ - (**XIII**, wherein Y, X and $R^{10}$ have the previously defined meaning) in the presence of a base such as triethylamine in a suitable solvent such as compound **XIII** itself or chloroform, or alternatively, reacting **VI** with a compound of general formula HO-C(=X)-$R^{10}$ (**XIV**, wherein X and $R^{10}$ have the previously defined meaning) in the presence of a dehydrating agent such as diciclohexylcarbodiimide in a polar solvent such as dimethylformamide;

(c) in all cases wherein X is sulphur, said compounds may also be obtained by reacting a compound of formula **I** wherein X is oxygen with a thiation reagent such as Lawesson's reagent in a suitable solvent such as toluene;

(d) in all cases wherein $R^5$ is acetoxy and $R^6$ is hydrogen, reacting a compound of formula **I** wherein $R^5$ is hydroxyl and $R^6$ is hydrogen with acetic anhydride in the presence of a base such as pyridine;

(e) in all cases wherein $R^5$ is formyloxy and $R^6$ is hydrogen, reacting a compound of formula **I** wherein $R^5$ is hydroxyl and $R^6$ is hydrogen with formic acid in the presence of a base such as pyridine;

(f) in all cases wherein $R^5$ and $R^6$ together form a bond, reacting a compound of general formula **I** wherein $R^5$ is OH and $R^6$ is hydrogen with a base such as sodium hydride or sodium hydroxyde in a suitable solvent such as toluene or dioxane; or reacting a compound of general formula **I** wherein $R^5$ is acetoxy and $R^6$ is hydrogen with a base such as 1,8-diazabicyclo(5.4.0)undec-7-ene (DBU) in an inert solvent such as toluene;

(g) optionally, interconverting the groups $R^1$, $R^2$, $R^{1'}$ and/or $R^{2'}$ in a compound of formula **I** or any intermediate of formula **II-VI** into other groups $R^1$ and/or $R^2$;

(h) and optionally, reacting a compound of formula **I** with an acid to give its corresponding acid addition salt.

**2.** A process according to Claim 1 in which the reagents and reaction conditions are so chosen as to prepare a compound of formula **Ia**

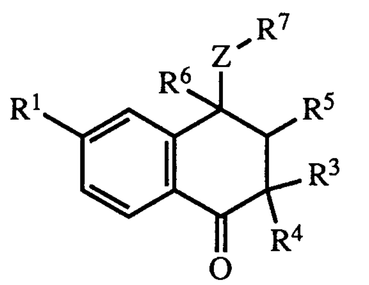

**Ia**

wherein $R^1$, $R^3$, $R^4$, $R^5$, $R^6$, $R^7$ and Z have the previously defined meaning.

**3.** A process according to Claim 1 in which the reagents and reaction conditions are so chosen as to prepare a compound of formula **Ib**

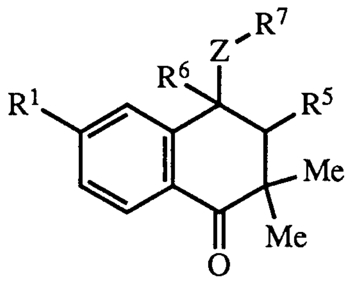

**Ib**

wherein $R^1$, $R^5$, $R^6$, $R^7$ and Z have the previously defined meaning.

**4.** A process according to Claim 1 in which the reagents and reaction conditions are so chosen as to prepare a compound of formula **Ic**

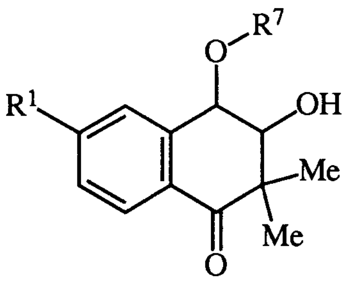

**Ic**

wherein $R^1$ and $R^7$ have the previously defined meaning.

**5.** A process according to Claim 1 in which the reagents and reaction conditions are so chosen as to prepare a compound of formula **Id**

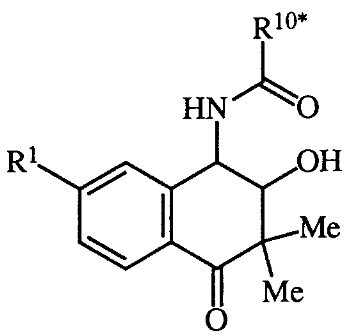

**Id**

wherein $R^1$ has the previously defined meaning, and $R^{10*}$ means phenyl or heteroaryl, both optionally substituted by a group chosen from halogen, $C_{1-6}$ alkyl, $C_{1-6}$ alkoxy, $C_{1-6}$ thioalkoxy, $C_{1-6}$ alkylcarbonyloxy, $C_{1-6}$ alkylcarbonyl, $C_{1-6}$ alkylthiocarbonyl, $C_{1-6}$ alkoxycarbonyl, hydroxy, oxo, nitro, cyano, trifluoromethyl, carboxyl, mercapto, amino or amino substituted by one or two $C_{1-4}$ alkyl or aryl groups.

6.

a) A process according to Claim 1 in which the reagents and reaction conditions are so chosen as to prepare *trans* -2,2-dimethyl-3-hydroxy-4-(6-hydroxy-3-pyridazinyloxy)-1-oxo-1,2,3,4-tetrahydronaphthalen-6-carbonitrile and the salts thereof.

b) A process according to Claim 1 in which the reagents and reaction conditions are so chosen as to prepare *rac-trans*-2,2-dimethyl-3-hydroxy-4-(6-hydroxy-1-methyl-3-pyridazinyloxy)-1-oxo-1,2,3,4-tetrahydronaphthalen-6-carbonitrile and the salts thereof.

c) A process according to Claim 1 in which the reagents and reaction conditions are so chosen as to prepare *(-)-trans*-2,2-dimethyl-3-hydroxy-4-(6-hydroxy-1-methyl-3-pyridazinyloxy)-1-oxo-1,2,3,4-tetrahydronaphthalen-6-carbonitrile and the salts thereof.

d) A process according to Claim 1 in which the reagents and reaction conditions are so chosen as to prepare *trans*-2,2-dimethyl-3-hydroxy-4-(4-methoxyphenylcarbonylamino)-1-oxo-1,2,3,4-tetrahydronaphthalen-6-carbonitrile and the salts thereof.

European Patent Office

# EUROPEAN SEARCH REPORT

Application Number

EP 92 11 3007

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 368 160 (MERCK)<br>* claims *<br>--- | 1,2,7,8 | C07D237/16<br>C07D239/42<br>C07C255/56<br>C07C255/60<br>C07D213/64<br>C07D213/81<br>C07D307/68<br>A61K31/395 |
| A | EP-A-0 168 619 (BEECHAM)<br>* claims *<br>----- | 1,2,7,8 | |
| | | | TECHNICAL FIELDS SEARCHED (Int. Cl.5 ) |
| | | | C07D<br>C07C |

The present search report has been drawn up for all claims

| Place of search | Date of completion of the search | Examiner |
|---|---|---|
| THE HAGUE | 30 OCTOBER 1992 | FRANCOIS J.C. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document